# EUROPEAN PATENT APPLICATION

(11) **EP 3 695 823 A1**
(43) Date of publication of application: **19.08.2020**
(21) Application number: 18866553.3
(22) Date of filing: 01.10.2018
(51) Int. Cl.: A61J 1/05

(54) **DRUG STORAGE CONTAINER, CLOSING MEMBER, METHOD FOR MANUFACTURE OF DRUG STORAGE CONTAINER, METHOD FOR INSPECTION OF MICROORGANISMS AND CONTAMINANTS, AND SOLID PREPARATION FOR BUFFER SOLUTION PREPARATION**

(30) Priority: 10.10.2017 JP 2017197188
(71) Applicant: Seikagaku Corporation, Tokyo 100-0005 (JP)
(72) Inventor: WATANABE, Masanao, Tokyo 162-8001 (JP); ISHII, Makoto, Tokyo 162-8001 (JP); ODA, Toshio, Tokyo 100-0005 (JP); MIZUMURA, Hikaru, Tokyo 100-0005 (JP)
(74) Representative: Müller-Boré & Partner Patentanwälte PartG mbB
(86) International application number: PCT/JP2018/036736
(87) International publication number: WO 2019/073846

(57) **Abstract**

A main object of the present disclosure is to provide a drug storage container enabling simple and reliable preparation of a mixed solution containing a drug and an auxiliary agent, a closing member that is for use in the drug storage container and configured to detachably retain a solid preparation containing a drug or an auxiliary agent, a method for manufacture of the drug storage container, a microbial contaminant test method using the drug storage container, and a solid preparation for preparing a buffer solution.

## Description

### Technical Field

The present disclosure relates to a drug storage container that is used for preparation of a mixed solution containing a drug and an auxiliary agent. Further, the present disclosure relates to a closing member that is used in the drug storage container, a method for manufacture of the drug storage container, a microbial contaminant test method using the drug storage container, and a solid preparation for preparing a buffer solution.

### Background Art

In the preparation of a reagent solution to be used in a test for a microbial contaminant (hereinafter referred to as a reagent solution for a microbial contaminant test or a simply referred to as a reagent solution) or a medicine (for example, a biological drug of an injection formulation, or the like), in one embodiment, for example, an operation of preparing a mixed solution by putting a predetermined amount of an auxiliary agent in a liquid form (an auxiliary agent solution) into a container in which a desired amount of a drug in a solid form or a liquid form (a solid drug or a drug solution) is enclosed beforehand to dissolve or dilute the drug is carried out. The operation is specifically an operation of putting a solution containing an auxiliary agent such as a buffer or a solubilizing agent for adjusting the mixed solution to a pH suitable for the use or for assisting the dissolution of the solid drug. Further, in one embodiment of the microbial contaminant test, an operation in which the above-prepared mixed solution is used as a reagent solution, and an analyte to be subjected to a test for a microbial contaminant is put into the reagent solution is successively carried out.

In medicine and food hygiene management and diagnosis of animals including humans, a test for microbial contaminants is sometimes carried out for measuring the degree of microbial contamination. As a means for carrying out the test, a Limulus test has been spread. The Limulus test is a technique for measuring the degree of microbial contamination using an endotoxin or (1→3)-β-D-glucan as a measurement target, and is a measurement method for a microbial contaminant utilizing a property that a protease precursor (factor C, factor B, factor G, or proclotting enzyme) possessed by a horseshoe crab is sequentially activated by such a microbial contaminant. The Limulus test is carried out using a reagent containing all or part of the protease precursors (hereinafter referred to as "Limulus reagent") such as a blood cell extract of a horseshoe crab (Limulus amoebocyte lysate (LAL), hereinafter referred to as "lysate reagent").

A preparation method for the above mixed solution will be described below by showing an endotoxin test as an example. A reagent solution to be used in an endotoxin test is prepared through, for example, an operation of breaking the seal of a container (vial) enclosing a lyophilized Limulus reagent, an operation of weighing a required amount of an auxiliary agent solution containing a buffer or the like and putting the solution into the container, an operation of covering the container with a dry-heat sterilized aluminum foil, and an operation of dissolving the Limulus reagent by stirring the container with a test tube mixer.

In the endotoxin test, for example, thereafter, a sample to be subjected to measurement of an endotoxin is prepared through an operation of putting an analyte into the reagent solution, an operation of covering the container with a dry-heat sterilized aluminum cap, and an operation of mixing the Limulus reagent and the analyte in the reagent solution by stirring the container with a test tube mixer (NPL 1).

### Citation List

### Non Patent Literature

NPL 1: "Endospecy (registered trademark) ES-24S Set package insert" Seikagaku Corporation

### Summary of Invention

### Technical Problem

In one embodiment of a test method for a microbial contaminant, it is necessary to perform a plurality of operations for preparation of a reagent solution including a Limulus reagent and preparation of a sample to be subjected to measurement of a microbial contaminant (hereinafter sometimes simply referred to as "sample for measurement"), and therefore, the preparation is complicated and requires much time and labor. Accordingly, it has been understood by some skilled in the art that the test method of the above embodiment is not suitable for immediately carrying out a test for a microbial contaminant when needed, and reduction of the operation steps has been demanded. Further, for example, in the above embodiment, when an incorrect operation of putting distilled water packed together in a kit for measurement in mistake for an auxiliary agent solution (buffer solution) is performed, a protease precursor contained in a Limulus reagent is not sufficiently activated by a microbial contaminant, and a problem of making an erroneous false-negative decision may occur. Also from the above point of view, the above embodiment of the test has a problem to be solved.

An operation of preparing the above auxiliary agent solution in the preparation of the mixed solution is an operation that is complicated and requires much time and labor. Further, it is necessary to prepare a plurality of auxiliary agent solutions according to the type of a drug, and therefore, not only does it require much time and labor for storage management, but also a problem of taking a wrong auxiliary agent solution when preparing the mixed solution or the like may occur. Therefore, a problem that the mixed solution cannot be immediately prepared or used when needed, the mixed solution prepared by taking a wrong auxiliary agent solution does not exhibit a desired effect, or the like may occur. An auxiliary agent solution having been prepared by adjusting the concentration or the like beforehand and enclosing it in an ampule tube or the like has also been made into a product, however, even in the case of using it, the problem of requiring much time and labor for storage management, taking a wrong auxiliary agent solution, or the like still remains. In particular, an injection formulation to be used for a treatment of animals including humans not only does not exhibit a desired drug efficacy, but also may harm the body when a mistake is made in the preparation operation. Also from such a point of view, a simpler and more reliable preparation method for the mixed solution has been demanded.

In order to omit the operation of preparing the auxiliary agent solution and storage management thereof, for example, a method for enclosing a lyophilizate of a mixed solution containing a drug and an auxiliary agent in a container body beforehand is also contemplated. This is because according to the above method, a desired mixed solution can be obtained only by an operation of putting a predetermined amount of an aqueous solvent into the container body. However, the above method has a problem that the storage stability of the drug may be deteriorated depending on the combination of the drug and the auxiliary agent, it is difficult to obtain a lyophilizate of the drug when an auxiliary agent containing a highly sublimable component is used, or the like, and it is difficult to say that the method is an appropriate method. For example, when a mixed solution of a Limulus reagent and a buffer is lyophilized, due to an extremely small amount of a microbial contaminant that may be mixed in the manufacture step, activation of the protease precursor proceeds before a microbial contaminant test is carried out, and as a result, a problem of making an erroneous false-positive decision may occur when measuring an analyte.

The present disclosure has been made in view of the above problems, and provides a means for simply and reliably carrying out preparation of a mixed solution containing a drug and an auxiliary agent while suppressing deterioration of the storage stability of the drug without requiring preparation of an auxiliary agent solution and storage management thereof. Further, the present disclosure provides a means for simply and reliably carrying out a test for a microbial contaminant. Still further, the present disclosure provides a means for easily carrying out pH adjustment for the mixed solution or a sample using the same.

That is, a main object of the present disclosure is to provide a drug storage container enabling simple and reliable preparation of a mixed solution containing a drug and an auxiliary agent, a closing member that is used in the drug storage container and detachably retains a solid preparation containing a drug or an auxiliary agent, a method for manufacture of the drug storage container, a microbial contaminant test method using the drug storage container, and a solid preparation for preparing a buffer solution.

### Solution to Problem

### <1> Drug Storage Container

One embodiment of the present disclosure provides a drug storage container, including: a container body having an opening portion at one end; and a closing member that closes the opening portion of the container body, wherein in the container body with the opening portion closed by the closing member, a drug and an auxiliary agent exist separately (hereinafter sometimes referred to as the drug storage container of the present invention).

According to the drug storage container of the present invention, in the container body with the opening portion closed by the closing member (that is, in a closed space), a drug and an auxiliary agent exist separately, and by mixing both substances in the coexistence with an aqueous solvent such as water or an aqueous solution, the drug and the auxiliary agent are dissolved or diluted, whereby a mixed solution can be prepared. Therefore, the time and labor associated with preparation of an auxiliary agent solution and storage management thereof when preparing the mixed solution containing the drug and the auxiliary agent can be omitted. Further, an operation of putting an auxiliary agent solution into the container body can be omitted, and therefore, an accident caused by taking a wrong auxiliary agent solution can be prevented. As a result, according to the drug storage container of the present invention, a mixed solution containing a drug and an auxiliary agent can be simply and reliably prepared before use in one mixing operation. Further, in the drug storage container of the present invention, the drug and the auxiliary agent exist separately in the closed space before preparing the mixed solution, and therefore, deterioration of the storage stability of the drug due to the existence of the auxiliary agent can be prevented.

In the above invention, the drug is not particularly limited. The drug may be a medicine such as a drug substance that is a pharmacologically active substance, or a formulation containing the same, and above all, the drug is preferably a protein or a protein formulation. In addition, in the above invention, the auxiliary agent is not particularly limited. The auxiliary agent may be a substance to be added to a drug substance for forming a pharmaceutical composition suitable for administration to animals including humans, and above all, the auxiliary agent is preferably an auxiliary agent containing at least one or more types selected from a solubilizing agent, an isotonizing agent, and a soothing agent as an auxiliary agent component. This is because by configuring the drug and the auxiliary agent to have the above compositions, a medicine such as a biological drug can be simply and reliably prepared using the drug storage container of the present invention.

Further, in the above invention, it is preferred that the auxiliary agent is an auxiliary agent containing a buffer as an auxiliary agent component. This is because by containing a buffer as an auxiliary agent component, pH adjustment for a medicine such as a biological drug becomes possible.

In the above invention, for example, the drug is preferably a Limulus reagent. Further, the auxiliary agent is preferably an auxiliary agent containing a buffer as an auxiliary agent component. This is because by configuring the drug and the auxiliary agent to have the above compositions, a reagent solution for a microbial contaminant test can be prepared using the drug storage container of the present invention. In addition, this is because after preparation of the reagent solution or at the same time of preparation of the mixed solution containing the drug and the auxiliary agent, preparation of a sample for measurement containing the drug and the auxiliary agent and an analyte can be simply and reliably carried out in the same closed space only by an operation of directly putting the analyte into the drug storage container of the present invention.

In the above invention, it is preferred that the auxiliary agent contains a buffer as an auxiliary agent component. In the above case, it is preferred that the auxiliary agent contains the buffer in such an amount that the pH becomes neutral when it is mixed with a predetermined aqueous solvent. This is because the pH of the obtained mixed solution can be made neutral in one operation of mixing the drug and the auxiliary agent, and therefore, a separate pH adjustment operation is no longer required, and the mixed solution can be used immediately after preparation.

In the above invention, it is preferred that the drug or the auxiliary agent is detachably retained on the closing member. At that time, the other substance of the drug and the auxiliary agent that is not retained on the closing member may exist on the inner wall or the bottom face in the container body without coming into contact with the closing member. This is because it becomes easy to allow the drug and the auxiliary agent to exist separately in the closed space, and by detaching the drug or the auxiliary agent retained on the closing member, preparation of the mixed solution can be simply carried out.

In the above invention, it is preferred that the form of the drug or the auxiliary agent is a solid preparation. Above all, it is preferred that the form of the drug or the auxiliary agent retained on the closing member is a solid preparation. This is because by adopting a solid preparation as the form, it becomes easy to retain the drug or the auxiliary agent on the closing member and detach the drug or the auxiliary agent from the closing member.

In the above invention, it is preferred that the closing member has a plug portion that is fitted into the opening portion of the container body, and the drug or the auxiliary agent is retained on the plug portion. This is because by fitting the plug portion inside the opening portion of the container body, the drug and the auxiliary agent can be stored in a sealed state.

Further, in the case of the above invention, it is preferred that the plug portion of the closing member has a recess portion on a face crossing the fit-in direction, and the drug or the auxiliary agent is retained on the recess portion. This is because by retaining the drug or the auxiliary agent on the recess portion of the plug portion, the drug or the auxiliary agent can be reliably retained.

### <2> Closing Member

One embodiment of the present disclosure provides a closing member, which is a closing member for use in the above drug storage container, being configured to detachably retain the solid preparation containing the drug or the auxiliary agent (hereinafter sometimes referred to as the closing member of the present invention).

According to the closing member of the present invention, when the closing member of the present invention is used in the above drug storage container, even if another substance, which is contraindicated or not preferred for premixing, exists in the container body, it can be made to exist individually separately at a stage before preparation of a mixed solution. Further, the closing member detachably retains the solid preparation, and therefore, by detaching the solid preparation and putting an aqueous solvent as needed, a mixed solution with another substance existing in the container body can be prepared before use, and for example, a mixed solution containing a drug or an auxiliary agent having poor storage stability in an aqueous solvent can be simply prepared.

### <3> Method for Manufacture of Drug Storage Container

One embodiment of the present disclosure provides a method for manufacture of a drug storage container, which is a method for manufacture of the above drug storage container, including a putting step of putting the drug through the opening portion of the container body, and a closing step of closing the container body in a state where the auxiliary agent is retained on the closing member while placing a face of the closing member on which the auxiliary agent is retained at the opening portion side of the container body, or including a putting step of putting the auxiliary agent through the opening portion of the container body, and a closing step of closing the container body in a state where the drug is retained on the closing member while placing a face of the closing member on which the drug is retained at the opening portion side of the container body (hereinafter sometimes referred to as the first embodiment of the method for manufacture of a drug storage container of the present invention).

According to the first embodiment of the method for manufacture of a drug storage container of the present invention, a drug storage container in which a drug and an auxiliary agent exist separately in the container body with the opening portion of the container body closed by the closing member (in a closed space) can be easily obtained.

Further, one embodiment of the present disclosure provides a method for manufacture of a drug storage container, which is a method for manufacture of the above drug storage container, including a putting step of putting the drug in a liquid form through the opening portion of the container body, a closing step of closing the container body in a state where the auxiliary agent is retained on the closing member while placing a face of the closing member on which the auxiliary agent is retained at the opening portion side of the container body, and a drying step of lyophilizing the drug in a liquid form in the container body so that the drug is solidified, or including a putting step of putting the auxiliary agent in a liquid form through the opening portion of the container body, a closing step of closing the container body in a state where the drug is retained on the closing member while placing a face of the closing member on which the drug is retained at the opening portion side of the container body, and a drying step of lyophilizing the auxiliary agent in a liquid form in the container body so that the auxiliary agent is solidified (hereinafter sometimes referred to as the second embodiment of the method for manufacture of a drug storage container of the present invention).

According to the second embodiment of the method for manufacture of a drug storage container of the present invention, contamination of the auxiliary agent and the drug can be reliably prevented by lyophilizing the auxiliary agent or the drug in a liquid form in the container body in a state where the opening portion of the container body is closed by the closing member in which the drug or the auxiliary agent is retained. Accordingly, a drug storage container in which a drug and an auxiliary agent exist separately can be easily obtained. Further, by performing lyophilization by the above method, deterioration of the storage stability of the drug before preparing a mixed solution can be prevented.

Further, one embodiment of the present disclosure provides a method for manufacture of a drug storage container, which is a method for manufacture of the above drug storage container, including a putting step of putting the drug in a liquid form through the opening portion of the container body, a closing step of closing the container body by temporarily fitting the plug portion of the closing member to the opening portion of the container body in a state where the auxiliary agent is retained on the plug portion of the closing member, and a drying step of lyophilizing the drug in a liquid form in the container body so that the drug is solidified in a temporarily fitted state, or including a putting step of putting the auxiliary agent in a liquid form through the opening portion of the container body, a closing step of closing the container body by temporarily fitting the plug portion of the closing member to the opening portion of the container body in a state where the drug is retained on the plug portion of the closing member, and a drying step of lyophilizing the auxiliary agent in a liquid form in the container body so that the auxiliary agent is solidified in a temporarily fitted state (hereinafter sometimes referred to as the third embodiment of the method for manufacture of a drug storage container of the present invention).

According to the third embodiment of the method for manufacture of a drug storage container of the present invention, the drug or the auxiliary agent is retained on the plug portion of the closing member, and contamination of the auxiliary agent and the drug can be reliably prevented by lyophilizing the auxiliary agent or the drug in a liquid form in the container body in a state where the plug portion is temporarily fitted to the opening portion of the container body. Accordingly, a drug storage container in which a drug and an auxiliary agent exist separately can be easily obtained. In addition, by performing lyophilization by the above method, deterioration of the storage stability of the drug before preparing a mixed solution can be prevented. Further, since the opening portion of the container body is temporarily fitted with the plug portion, air in the container body can be easily removed from a gap at the temporary fitting position in the drying step, and therefore, lyophilization can be efficiently carried out, and also contamination with an impurity from the outside can be prevented.

In the case of the above intention, it is preferred that the plug portion of the closing member has the recess portion on a face crossing the fit-in direction, the recess portion of the plug portion has an enough depth to expose a part of the recess portion through the opening portion of the container body when the plug portion is temporarily fitted to the opening portion of the container body in the closing step, and the drug or the auxiliary agent is retained on the recess portion of the plug portion. This is because by exposing a part of the recess portion of the plug portion through the opening portion of the container body, in the drying step, the exposed part of the recess portion functions as a degassing channel for air in the container body, and lyophilization can be more efficiently carried out.

### <4> Microbial Contaminant Test Method

One embodiment of the present disclosure provides a microbial contaminant test method, which is a microbial contaminant test method for testing a microbial contaminant using the above-mentioned drug storage container, including a preparation step of preparing a sample that contains the drug and the auxiliary agent and an analyte and is subjected to measurement of a microbial contaminant (sample for measurement) by putting the analyte into the container body of the drug storage container, and a detection step of detecting the microbial contaminant in the sample (hereinafter sometimes referred to as the microbial contaminant test method of the present invention).

According to the microbial contaminant test method of the present invention, by putting an analyte into the container body of the above-mentioned drug storage container, a sample for measurement containing a drug, an auxiliary agent, and an analyte can be prepared collectively in the container body. That is, according to the present invention, the sample for measurement can be easily prepared in one step without requiring a step of preparing and weighing an auxiliary agent solution, a step of preparing a reagent solution by putting the auxiliary agent solution into the container body containing a drug, or the like. Then, detection of a microbial contaminant can be carried out using the drug storage container in which preparation of the sample for measurement has been carried out. In such a manner, according to the microbial contaminant test method of the present invention, preparation of a sample for measurement can be easily carried out, and determination of the presence or absence of a microbial contaminant in an analyte contained in the sample for measurement or measurement of the amount thereof can be simply and reliably carried out, and the degree of microbial contamination can be simply measured.

In the above intention, the microbial contaminant is a component derived from a microorganism (a nucleic acid, a protein, a lipid, a carbohydrate such as a saccharide, or the like), and means a component (contaminant) other than the constituent components of the drug and the auxiliary agent, and the analyte itself. In the above intention, it is preferred that the microbial contaminant is an endotoxin or (1→3)-β-D-glucan.

### <5> Solid preparation for preparing Buffer Solution

One embodiment of the present disclosure provides a solid preparation for preparing a buffer solution, including at least a buffer and a molding agent, wherein the buffer is contained in such an amount that the pH becomes neutral when the solid preparation is mixed with a predetermined aqueous solvent (hereinafter sometimes referred to as the solid preparation for preparing a buffer solution of the present invention).

According to the solid preparation for preparing a buffer solution of the present invention, the pH becomes neutral by mixing it with a predetermined aqueous solvent, and therefore, a buffer solution showing a predetermined pH can be easily adjusted. Further, by using the solid preparation for preparing a buffer solution of the present invention when preparing a mixed solution containing a drug and an auxiliary agent, and a sample using the mixed solution, adjustment of the pH of the mixed solution or the sample can be easily carried out.

In the above invention, it is preferred that the solid preparation does not substantially contain a microbial contaminant. This is because the solid preparation for preparing a buffer solution of the present invention can be used for preparation of a mixed solution intended to be administered to animals including humans as it is or preparation of a sample for measurement for a microbial contaminant test.

### Advantageous Effects of Invention

According to the present disclosure, a means for simply and reliably carrying out preparation of a mixed solution containing a drug and an auxiliary agent while suppressing deterioration of the storage stability of the drug without requiring preparation of an auxiliary agent solution can be provided. In addition, by using the means, a test method for a microbial contaminant enabling simple and reliable evaluation can be provided. Further, a solid preparation for preparing a buffer solution enabling easy adjustment of the pH of a mixed solution or a sample using the mixed solution can be provided.

### Brief Description of Drawings

[FIG. 1] FIG. 1 is a schematic cross-sectional view showing an example of a drug storage container of a first embodiment of the present disclosure.
[FIG. 2] FIG. 2 is a schematic perspective view and a plan view showing an example of a closing member of the first embodiment of the present disclosure.
[FIG. 3] FIG. 3 is a schematic perspective view showing another example of the drug storage container of the first embodiment of the present disclosure.
[FIG. 4] FIG. 4 is a schematic cross-sectional view showing other examples of the closing member of the first embodiment of the present disclosure, and an example of a mode of retaining a drug or an auxiliary agent.
[FIG. 5] FIG. 5 is a schematic cross-sectional view showing another example of the closing member of the first embodiment of the present disclosure, and an example of a mode of retaining a drug or an auxiliary agent.
[FIG. 6] FIG. 6 is a schematic cross-sectional view showing another example of the closing member of the first embodiment of the present disclosure.
[FIG. 7] FIG. 7 is a schematic plan view showing other examples of the closing member of the first embodiment of the present disclosure.
[FIG. 8] FIG. 8 is a schematic cross-sectional view showing another example of the closing member of the first embodiment of the present disclosure, and examples of a mode of retaining a drug or an auxiliary agent.
[FIG. 9] FIG. 9 is a schematic view illustrating a method for putting an aqueous solvent into the drug storage container of the first embodiment of the present disclosure.
[FIG. 10] FIG. 10 is a schematic perspective view showing another example of the closing member of the first embodiment of the present disclosure.
[FIG. 11] FIG. 11 is a schematic view showing an example of a usage of the drug storage container of the first embodiment of the present disclosure.
[FIG. 12] FIG. 12 is a process diagram showing an example of a method for manufacture of the drug storage container of the first embodiment of the present disclosure.

### Description of Embodiments

Hereinafter, a drug storage container, a closing member, a method for manufacture of a drug storage container, a test method for a microbial contaminant, and a solid preparation for preparing a buffer solution of a first embodiment of the present disclosure will be described in detail, respectively.

### I. Drug Storage Container

The drug storage container of the present invention includes a container body having an opening portion at one end, and a closing member that closes the opening portion of the container body, and in the container body with the opening portion closed by the closing member, a drug and an auxiliary agent exist separately.

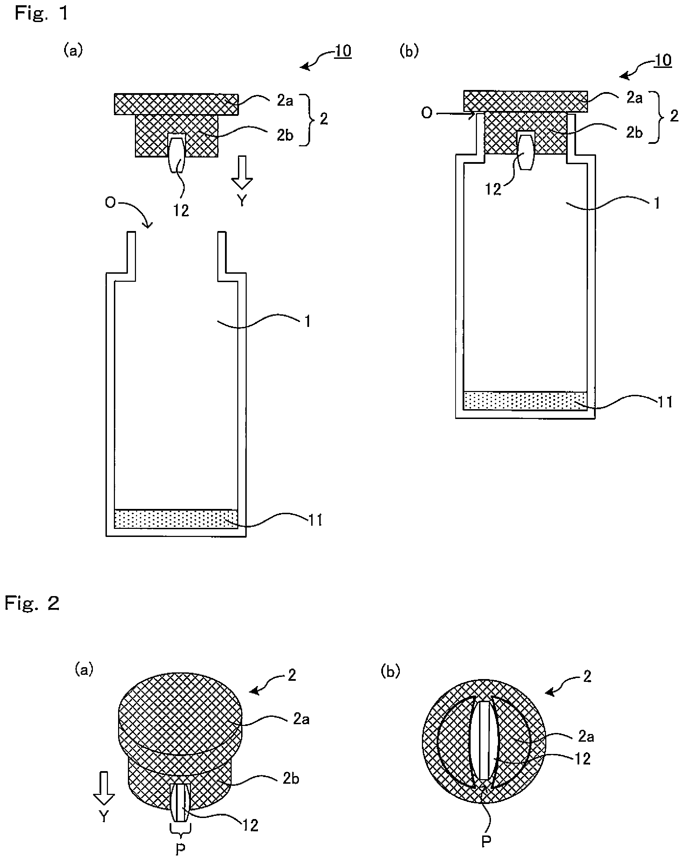

The drug storage container of the present invention will be described with reference to the drawings. FIGS. 1(a) and 1(b) are schematic cross-sectional views showing an example of the drug storage container of the present invention, and FIG. 1(a) shows the container before closing an opening portion by a closing member 2, and FIG. 1(b) shows the container after closing the opening portion by the closing member 2. Further, FIG. 2(a) shows a schematic perspective view of the closing member in the drug storage container shown in FIGS. 1(a) and 1(b), and FIG. 2(b) shows a schematic plan view seen from the plug portion side in FIG. 2(a). A drug storage container 10 shown in FIGS. 1(a) and 1(b) includes a container body 1 having an opening portion O at one end, and the closing member 2 that closes the opening portion O. The arrow Y in FIG. 1(a) indicates a fit-in direction Y when the closing member 2 is fitted to the opening portion O. As shown in FIG. 1(b), a drug 11 and an auxiliary agent 12 exist separately in the container body 1 with the opening portion O closed by the closing member 2 (in a closed space).

Further, in the examples shown in FIGS. 1 and 2, the auxiliary agent 12 is retained on the closing member 2. Specifically, the closing member 2 includes a top plate portion 2a and a plug portion 2b that is fitted into the opening portion O on a face of the top plate portion 2a at the container body 1 side, and a solid preparation (tablet) containing the auxiliary agent 12 is retained on the plug portion 2b. More specifically, as shown in FIGS. 2(a) and 2(b), in the plug portion 2b, a recess portion P is formed on a face crossing the fit-in direction Y, and the auxiliary agent 12 is pinched and retained on the recess portion P. The closing member 2 illustrated in FIGS. 1 and 2 is a sealing member enabling sealing by fitting the plug portion 2b inside the opening portion O of the container body 1.

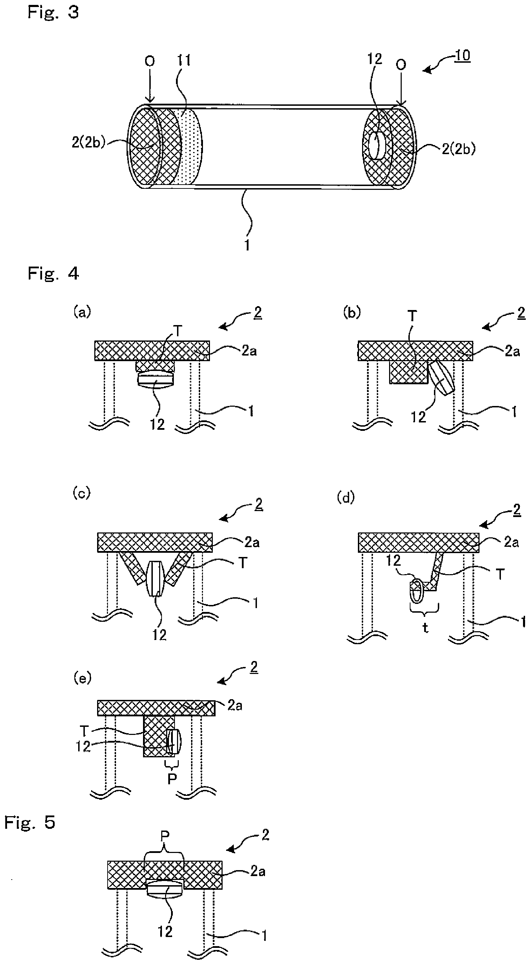

FIG. 3 is a schematic perspective view showing another example of the drug storage container of the present invention, and shows an example in which the container body 1 of the drug storage container 10 of the present invention is a syringe having the opening portion O at both ends. In the example shown in FIG. 3, the opening portions O at both ends of the container body 1 are closed by the closing members 2 that are different from each other, respectively. The entire closing member 2 becomes the plug portion 2b that is fitted into the opening portion O. In addition, in the container body 1 with the opening portions O closed by the closing members 2, the drug 11 and the auxiliary agent 12 exist separately. In the example shown in FIG. 3, the drug 11 and the auxiliary agent 12 are retained on the different closing members 2, respectively.

According to the drug storage container of the present invention, in the container body with the opening portion closed by the closing member (that is, in the closed space), a drug and an auxiliary agent exist separately, and by mixing both substances in the coexistence with an aqueous solvent such as water or an aqueous solution, the drug and the auxiliary agent are dissolved or diluted, whereby a mixed solution can be prepared. Therefore, the time and labor associated with preparation of an auxiliary agent solution and storage management thereof when preparing a mixed solution containing a drug and an auxiliary agent can be omitted. Further, an operation of putting an auxiliary agent solution into the container body can be omitted, and therefore, an accident caused by taking a wrong auxiliary agent solution can be prevented. As a result, according to the drug storage container of the present invention, a mixed solution containing a drug and an auxiliary agent can be simply and reliably prepared before use in one mixing operation.

Further, in the drug storage container of the present invention, the drug and the auxiliary agent exist separately in the closed space before preparing the mixed solution, and therefore, deterioration of the storage stability of the drug due to the existence of the auxiliary agent can be prevented.

Hereinafter, the drug storage container of the present invention will be described for each component.

### A. Drug and Auxiliary Agent

In the drug storage container of the present invention, the drug and the auxiliary agent exist separately in the container body with the opening portion closed by the closing member (in the closed space).

The clause that the drug and the auxiliary agent "exist separately" in the closed space means that the drug and the auxiliary agent do not exist in admixture, but each exists independently.

It is preferred that the drug or the auxiliary agent is detachably retained on the closing member, and it is more preferred that the auxiliary agent is detachably retained on the closing member. This is because by retaining one of the drug and the auxiliary agent on the closing member and allowing the other to exist in the container body, it becomes easy to allow the drug and the auxiliary agent to exist separately in the closed space, and deterioration of the storage stability of the drug due to the existence of the auxiliary agent can be prevented. In addition, this is because when preparing the mixed solution, by detaching the substance retained on the closing member, preparation of the mixed solution can be simply carried out. When the auxiliary agent is detachably retained on the closing member, the drug only needs to exist in the container body, and may also be retained in the container body.

Note that with respect to "detachably" and with respect to a specific embodiment in which the drug and the auxiliary agent exist separately in the closed space, detailed descriptions will be given later.

The drug and the auxiliary agent can be appropriately selected and combined according to the mixed solution to be prepared. For example, when a medicine such as a biological drug is prepared, the drug and the auxiliary agent are not particularly limited, but the drug may be a medicine such as a drug substance that is a pharmacologically active substance, or a formulation containing the same, and above all, the drug is preferably a protein or a protein formulation. At that time, the auxiliary agent may be a substance to be added to a drug substance for forming a pharmaceutical composition suitable for administration to animals including humans, and above all, the auxiliary agent is preferably an auxiliary agent containing at least one or more types selected from a solubilizing agent, an isotonizing agent, and a soothing agent as an auxiliary agent component or an auxiliary agent containing a buffer as an auxiliary agent component. This is because a medicine such as a biological drug can be simply and reliably prepared using the drug storage container of the present invention.

Further, when preparing a reagent solution for a microbial contaminant test or a sample for measurement, the drug is preferably a Limulus reagent, and the auxiliary agent is preferably an auxiliary agent containing a buffer as an auxiliary agent component. This is because the reagent solution or the sample for measurement can be simply and reliably prepared using the drug storage container of the present invention. In addition, this is because after preparation of the reagent solution or at the same time of preparation of the mixed solution containing the drug and the auxiliary agent, preparation of a sample for measurement can be simply and reliably carried out in the same closed space only by an operation of directly putting an analyte into the drug storage container of the present invention.

Hereinafter, the drug and the auxiliary agent will be described.

### 1. Drug

The drug is a substance exhibiting a main function of the mixed solution to be prepared using the drug storage container of the present invention, and contains at least one type of active ingredient.

The type of the active ingredient contained in the drug is not particularly limited, and for example, can be appropriately selected according to the mode of use of the mixed solution containing the drug. The active ingredient contained in the drug may be only one type or may be two or more types. Further, the amount of the active ingredient contained in the drug is not particularly limited, and for example, can be appropriately set according to the mode of use of the mixed solution containing the drug.

As the drug, for example, a drug substance of an active ingredient or a formulation containing the active ingredient can be used. The active ingredient in the present invention refers to, for example, a component having a pharmacological activity in the body of an animal including a human, a component that causes a physical or chemical change in another substance such as a microbial contaminant or the active ingredient itself when it comes into contact with such another substance, or the like. Further, the physical or chemical change as used herein includes binding, transfer, translocation, addition, detachment, degradation, cleavage, oxidation, reduction, labeling, color development, light emission, and the like.

Examples of the drug in the present invention include medicines used for diagnosis, therapy, treatment, prevention, or the like of diseases of animals including humans, and reagents used for tests including clinical laboratory tests, examinations, researches, and the like.

### (1) Medicine

As the medicine, for example, a drug substance that is a pharmacologically active substance is exemplified. As the drug substance that is a pharmacologically active substance, for example, a protein is exemplified. Further, as the medicine, for example, a formulation containing a pharmacologically active substance as an active ingredient is exemplified. As the formulation containing a pharmacologically active substance, for example, a protein formulation containing a protein as an active ingredient is exemplified. The protein formulation can be the same as a known one, and examples thereof include collagen, serum albumin, fibrinogen, various hormones, erythropoietin, interferons, and interleukins. In addition, as the drug substance that is a pharmacologically active substance, a substance whose storage is difficult in a liquid form, and which is stored in a lyophilized form among vaccines, antibody pharmaceutical preparations (monoclonal antibodies against cytokines/tumor antigens/various receptors, or the like) is exemplified.

### (2) Reagent

As the reagent, for example, a Limulus reagent is exemplified. The Limulus reagent may be a blood cell extract of a horseshoe crab (lysate reagent) itself, and may be a material obtained by subjecting the blood cell extract to fractionation and/or purification, or the like as appropriate. In addition, the Limulus reagent may be a material obtained according to a conventional method using blood cells of a horseshoe crab as a raw material.

The species of the horseshoe crab is not particularly limited, and for example, it may be an arbitrary horseshoe crab belonging to the order of Xiphosura. As the horseshoe crab, a horseshoe crab belonging to the family Limulidae is exemplified. The horseshoe crab is preferably a horseshoe crab belonging to the genus Tachypleus, the genus Limulus, or the genus Carcinoscorpius. As the horseshoe crab, specifically, Tachypleus tridentatus, Tachypleus gigas, Limulus polyphemus, and Carcinoscorpius rotundicauda are exemplified.

As the Limulus reagent, for example, a reagent containing one or more types of Limulus factors including a Limulus factor that reacts with a microbial contaminant to be used as a measurement target substance in a Limulus test among respective factors (factor C, factor B, factor G, and proclotting enzyme, hereinafter individually or collectively referred to as "Limulus factor") that are protease precursors involved in a coagulation cascade reaction of a horseshoe crab (hereinafter simply referred to as "cascade reaction") is exemplified. The Limulus reagent may be, for example, a Limulus reagent that is artificially reconstructed so as to include only an arbitrary Limulus factor (hereinafter referred to as "reconstructed Limulus reagent"). The "reaction with a microbial contaminant" as used herein means a reaction in which a precursor of the Limulus factor coming into contact with a microbial contaminant is activated and changed so as to have protease activity.

When, for example, an endotoxin is determined to be a measurement target substance as a microbial contaminant, the reconstructed Limulus reagent only needs to contain at least factor C as the Limulus factor, and may or may not contain other Limulus factors (factor B, factor G, and proclotting enzyme) . Further, when, for example, (1→3)-β-D-glucan is determined to be a measurement target substance as a microbial contaminant, the reconstructed Limulus reagent only needs to contain at least factor G as the Limulus factor, and may or may not contain other Limulus factors (factor C, factor B, and proclotting enzyme).

When an endotoxin is determined to be a measurement target substance as a microbial contaminant, the reconstructed Limulus reagent preferably contains factor C as the Limulus factor, more preferably contains factor B other than factor C, and further more preferably contains factor B and proclotting enzyme other than factor C. In addition, when an endotoxin is determined to be a measurement target substance as a microbial contaminant, the reconstructed Limulus reagent preferably does not contain factor G.

When (1→3)-β-D-glucan is determined to be a measurement target substance as a microbial contaminant, the reconstructed Limulus reagent preferably contains factor G as the Limulus factor, and more preferably contains proclotting enzyme other than factor G. In addition, when (1→3)-β-D-glucan is determined to be a measurement target substance as a microbial contaminant, the reconstructed Limulus reagent preferably does not contain factor C, and more preferably does not contain factor C and factor B.

The reconstructed Limulus reagent can be prepared by, for example, performing purification or fractionation so as to remove an arbitrary Limulus factor from the Limulus reagent. Further, for example, one type of Limulus factor isolated from the Limulus reagent may be used as the reconstructed Limulus reagent or a mixture obtained by mixing two or more types of isolated Limulus factors may be used as the reconstructed Limulus reagent.

The preparation of the reconstructed Limulus reagent can be carried out by appropriately combining known methods, and for example, can be carried out with reference to the method disclosed in "Nakamura T, Horiuchi T, Morita T, Iwanaga S. J Biochem. 1986 Mar; 99(3): 847-57".

The Limulus factor contained in the reconstructed Limulus reagent may be a natural Limulus factor obtained from a horseshoe crab or may be a recombinant Limulus factor obtained by a genetic engineering technique.

The natural Limulus factor can be, for example, obtained from a blood cell extract of a horseshoe crab as described above. Further, the recombinant Limulus factor can be, for example, obtained by allowing a host cell transformed using a nucleic acid encoding a Limulus factor to express the Limulus factor. The type of the host cell is not particularly limited, but may be, for example, a mammalian cell or an insect cell. The host cell is preferably a mammalian cell. This is because a reconstructed Limulus reagent that is less susceptible to reaction inhibition due to the presence of a salt (ion) can be provided (WO 2014/92079). Examples of the mammalian cell include a Chinese hamster ovary-derived cell line (CHO cell) and a human embryonic kidney cell-derived cell line (HEK cell) . Examples of the HEK cell include an HEK 293 cell.

The expression of the recombinant Limulus factor by the host cell can be carried out according to a conventional method, and for example, can be carried out with reference to the method disclosed in WO 2014/92079. The amino acid sequence of the Limulus factor or the base sequence of a gene encoding the amino acid sequence can be obtained from a public database. Examples of the public database include a database provided by National Center for Biotechnology Information (NCBI). As for the expressed recombinant Limulus factor, for example, a culture solution obtained by culturing the host cell may be used as the Limulus factor as it is, or a material purified to a desired extent as needed may be used as the Limulus factor.

In the Limulus factors contained in the reconstructed Limulus reagent, all the Limulus factors may be natural Limulus factors or all the Limulus factors may be recombinant Limulus factors, or may be a material obtained by appropriately combining a natural Limulus factor and a recombinant Limulus factor. Further, the reconstructed Limulus reagent may be, for example, a Limulus reagent itself, or one obtained by appropriately subjecting a Limulus reagent to fractionation and/or purification, or the like, or a material obtained by appropriately combining natural and/or recombinant Limulus factors.

The Limulus reagent described above may be a commercially available Limulus reagent. Examples of the commercially available Limulus reagent include PyroSmart (Seikagaku Corporation), Endospecy (Seikagaku Corporation), Pyrochrome (Associates of Cape Cod, Inc.), Pyrotell-T (Associates of Cape Cod, Inc.), Pyrotell Single Test (Associates of Cape Cod, Inc.), Limulus ES-II Single Test (Wako Pure Chemical Industries, Ltd.), Kinetic-QCL (Lonza Walkersville, Inc.), and Endochrome-K (Charles River Laboratories, Inc.). These can be used as the drug in the present invention.

### (3) Others

The drug may be constituted by the above-mentioned active ingredient of the medicine or the reagent alone, or may be constituted by a composition containing the active ingredient and another component.

For example, the Limulus reagent may be a reagent constituted only by the Limulus factor that is the active ingredient, or may be a reagent containing the Limulus factor and another component. Examples of such another component include components other than the Limulus factor possessed by the host cell that expresses the Limulus factor or the horseshoe crab (specifically, a nucleic acid, a protein, a lipid, a carbohydrate such as a saccharide, and the like) . In addition, the Limulus reagent may contain a substrate for detection to be used in a Limulus test as the above-mentioned another component. The substrate for detection will be described later.

The mass concentration (w/w%) of the active ingredient contained in the drug may be, for example, 0.01 w/w% or more, 0.1 w/w% or more, 1 w/w% or more, 10 w/w% or more, 25 w/w% or more, 50 w/w% or more, 75 w/w% or more, 90 w/w% or more, 95 w/w% or more, 99 w/w% or more, or 100 w/w%. In addition, the mass concentration (w/w%) of the active ingredient contained in the drug may be, for example, 99.9 w/w% or less, 99 w/w% or less, 95 w/w% or less, 90 w/w% or less, 75 w/w% or less, 50 w/w% or less, 25 w/w% or less, 10 w/w% or less, or 1 w/w% or less.

Note that "w/w%" has the same meaning as a ratio calculated by "a predetermined material (component) (g) / the entire amount (g)". The same shall apply hereinafter.

### 2. Auxiliary Agent

The auxiliary agent is a substance containing one or more types of auxiliary agent components that impart another function other than the function of the drug in the mixed solution containing the drug.

The type of the auxiliary agent component contained in the auxiliary agent is not particularly limited, and can be appropriately selected according to the mode of use of the mixed solution containing the auxiliary agent, the type of the drug to be used, or the like. The auxiliary agent component contained in the auxiliary agent may be only one type or two or more types. In addition, the amount of the auxiliary agent component contained in the auxiliary agent is not particularly limited, and, for example, can be appropriately set according to the mode of use of the mixed solution containing the auxiliary agent.

When the drug is a medicine, the auxiliary agent preferably contains an auxiliary agent component to be added to a drug substance for forming a pharmaceutical composition suitable for administration to animals including humans, and preferably contains at least one or more types selected from a solubilizing agent, an isotonizing agent, and a soothing agent as the auxiliary agent component. As the solubilizing agent, the isotonizing agent, and the soothing agent, the same materials as used in a known biological drug can be used.

Further, in the above case, the auxiliary agent may contain a buffer, a diluent, a diuretic, an antibiotic, a nutrient, an antioxidant, or the like as the auxiliary agent component. Above all, the auxiliary agent preferably contains a buffer as the auxiliary agent component. This is because pH adjustment for a medicine such as a biological drug obtained using the drug storage container of the present invention becomes possible. The type of the buffer and the content of the buffer in the auxiliary agent will be described later.

When the drug is a reagent, the auxiliary agent preferably contains a buffer as the auxiliary agent component. This is because pH adjustment for the reagent solution or the sample for measurement becomes possible. In addition, the auxiliary agent may contain a substrate for detection to be used in a Limulus test or the like other than the auxiliary agent component. The substrate for detection will be described later.

Here, as the buffer, for example, citric acid, malic acid, lactic acid, ascorbic acid, maleic acid, gluconic acid, phosphoric acid, boric acid, amino acids such as glycine and glutamic acid, tris(hydroxymethyl)aminomethane (Tris), 2-[4-(2-hydroxyethyl)-1-piperazinyl]ethanesulfonic acid (HEPES), N-tris(hydroxymethyl)methyl-2-aminoethanesulfonic acid (TES), 2-hydroxy-3-[4-(2-hydroxyethyl)-1-piperazinyl]propanesulfo nic acid, monohydrate (HEPPSO), N-[tris(hydroxymethyl)methyl]glycine (Tricine), N,N-bis(2-hydroxy)ethyl)glycine (Bicine), imidazole, a phosphate, and the like are exemplified.

The content of the buffer in the auxiliary agent can be appropriately set according to the type or amount of the mixed solution to be prepared. Above all, the auxiliary agent preferably contains the buffer in such an amount that the pH becomes neutral when it is mixed with a predetermined aqueous solvent. This is because the pH of the obtained mixed solution can be made neutral in one operation of mixing the drug and the auxiliary agent, and therefore, a separate pH adjustment operation is no longer required, and the mixed solution can be used immediately after preparation. For example, when a medicine such as a biological drug is prepared, the medicine can be adjusted to a pH suitable for administration thereof to animals including humans. In addition, for example, when a reagent solution for a microbial contaminant test or a sample for measurement is prepared, it can be adjusted to a pH suitable for allowing a reaction in which a protease precursor (Limulus factor) is activated by a microbial contaminant or the cascade reaction to preferably proceed.

Here, the content of the buffer in the auxiliary agent may be such an amount that the pH becomes neutral according to the type or amount of the drug or the aqueous solvent in consideration of the buffer capacity or the like of such a substance.

The aqueous solvent to be mixed with the drug and the auxiliary agent is not particularly limited. Here, the aqueous solvent means a solvent miscible with water. As the "aqueous solvent", for example, water such as water for injection, an aqueous solution containing water and another component, an analyte in a liquid form, and the like are exemplified. In addition, as the "another component", for example, an inorganic component such as sodium chloride and an organic component such as a surfactant are exemplified. As the "analyte in a liquid form", for example, an analyte to be subjected to a Limulus test is exemplified, and specifically, water for injection, an injection, a sample derived from an animal including a human, and the like are exemplified. As the "sample derived from an animal", blood, serum, plasma, and the like are exemplified. As the "sample derived from an animal", a sample obtained by recovering a liquid directly or indirectly brought into contact with the body of an animal (a dialysis solution or the like) is also exemplified.

The aqueous solvent may contain an organic solvent, or may not substantially contain an organic solvent or may not contain an organic solvent at all. The phrase "not substantially contain an organic solvent" as used herein means that even if an organic solvent is contained, it is contained in such an amount that the target effect can be exhibited without losing the function of the drug in the mixed solution, and the volume concentration (v/v%) thereof may be, for example, within a range of more than 0 v/v% and 20 v/v% or less, within a range of more than 0 v/v% and 10 v/v% or less, within a range of more than 0 v/v% and 5 v/v% or less, within a range of more than 0 v/v% and 2 v/v% or less, or within a range of more than 0 v/v% and 1 v/v% or less.

The amount of the aqueous solvent to be mixed with the auxiliary agent is not particularly limited. The amount of the aqueous solvent to be mixed with the auxiliary agent can be set to such an amount that the pH becomes neutral without requiring dilution when it is mixed with the auxiliary agent, and for example, may be 0.1 mL or more or 0.2 mL or more. In addition, the amount of the aqueous solvent to be mixed with the auxiliary agent may be, for example, 1 L or less, 100 mL or less, 10 mL or less, 1 mL or less, or 0.5 mL or less. Therefore, as the range of the amount of the aqueous solvent to be mixed with the auxiliary agent, a range within a range of 0.1 mL to 1 L, within a range of 0.1 mL to 100 mL, within a range of 0.1 mL to 10 mL, within a range of 0.1 mL to 1 mL, within a range of 0.2 mL to 1 mL, within a range of 0.2 mL to 0.5 mL, or the like is exemplified. When the drug is a Limulus reagent, the range of the amount of the aqueous solvent to be mixed with the auxiliary agent is preferably, for example, within a range of 0.1 mL to 1 mL, more preferably within a range of 0.2 mL to 0.5 mL.

The clause that the pH is neutral in the above description means that the pH is neutral when it is measured at room temperature (24 to 26°C) . For example, the pH that is neutral at 25°C may be, for example, 6 or higher, 6.2 or higher, 6.4 or higher, 6.6 or higher, or 6.8 or higher. Further, the pH that is neutral may be, for example, 8 or less, 7.9 or less, 7.8 or less, 7.6 or less, or 7.4 or less. That is, as the range of the pH that is neutral, a range within a range of 6 to 8, within a range of 6.4 to 8, within a range of 6.8 to 8, within a range of 6.8 to 7.9, within a range of 6.4 to 7.8, within a range of 6.8 to 7.8, within a range of 6.4 to 7.4, within a range of 6.8 to 7.4, or the like is exemplified. When the drug is a Limulus reagent, the pH when the auxiliary agent containing the buffer as he auxiliary agent component is mixed with the predetermined aqueous solvent is preferably within a range of 6.8 to 7.9. The pH means a pH exhibited when the drug and the auxiliary agent and the predetermined aqueous solvent are mixed and the drug and the auxiliary agent are dissolved therein.

The content of the buffer in the auxiliary agent can also be defined by the molar concentration of the buffer (hereinafter referred to as "the final concentration of the buffer") when mixing the drug and the auxiliary agent and the predetermined aqueous solvent. The amount that "the pH becomes neutral when the auxiliary agent is mixed with the predetermined aqueous solvent" may be such an amount that the final concentration of the buffer when the auxiliary agent is mixed with the drug and the predetermined aqueous solvent falls within a predetermined range. Specifically, the final concentration of the buffer may be, for example, 1 mM or more, 5 mM or more, 10 mM or more, or 20 mM or more. The final concentration of the buffer may be, for example, 1 M or less, 500 mM or less, 250 mM or less, or 100 mM or less. Therefore, as the range of the final concentration of the buffer, a range within a range of 1 mM to 1 M, within a range of 1 mM to 500 mM, within a range of 1 mM to 250 mM, within a range of 1 mM to 100 mM, within a range of 5 mM to 500 mM, within a range of 5 mM to 250 mM, within a range of 5 mM to 100 mM, within a range of 10 mM to 500 mM, within a range of 10 mM to 250 mM, within a range of 10 mM to 100 mM, within a range of 20 mM to 250 mM, within a range of 20 mM to 100 mM, or the like is exemplified. When the drug is a Limulus reagent, the final concentration of the buffer is preferably within a range of 20 mM to 100 mM. Note that mM = mmol/L.

### 3. Another Component

In the drug and the auxiliary agent, another component may be contained. Hereinafter, another component will be described.

### (1) Substrate for Detection

At least one of the drug and the auxiliary agent may contain a substrate for detection (hereinafter simply referred to as "substrate"). The substrate is a component to be used in a Limulus test. The substrate is not particularly limited as long as it is a substance that can be used for determination of the presence or absence of an activated Limulus factor or measurement of the amount thereof, progress of the cascade reaction, or the like, and for example, a protein, a polypeptide, a peptide, and a derivative thereof are exemplified. The substrate may be a natural protein or the like or may be a recombinant protein or the like or may be a chemically synthesized protein or the like.

The substrate may be a substrate for detecting clotting enzyme that is the final product of the cascade reaction, or may be a substrate for detecting an activated Limulus factor (specifically, factor C, factor B, or factor G) that exists in a middle stage of the cascade reaction.

As the substrate, coagulogen serving as a substrate for the clotting enzyme that is the final product of the cascade reaction is exemplified. For example, natural coagulogen can be prepared from a blood cell extract of a horseshoe crab. Further, for example, recombinant coagulogen can be prepared, for example, with reference to the method described in "Miyata et al. , Protein, Nucleic Acid, and Enzyme, Separate Volume No. 29; pp. 30-43; 1986".

The substrate is preferably a derivative of a peptide (hereinafter referred to as "peptide substrate"). This is because the production cost is relatively low, and also the detection sensitivity is excellent. As the peptide substrate, a derivative of a peptide represented by a general formula: X-Y-Z (wherein X represents a peptide, Y represents a protecting group, and Z represents a labeling substance) is exemplified. In the peptide substrate, the protecting group (Y) is bonded through the α-amino group of an amino acid residue at the N terminus of the peptide (X), and the labeling substance (Z) is bonded through the carboxy group of an amino acid residue at the C terminus of the peptide (X). In the peptide substrate, specific modes of the respective constituent elements (X, Y, and Z) are not particularly limited as long as it has a property in which the bond between X and Z is cleaved by an activated Limulus factor to release the labeling substance (Z). The bond between X and Z in the peptide substrate is preferably an amide bond. In addition, in the above general formula, the protecting group (Y) may not exist, and as the peptide substrate, a derivative of a peptide represented by a general formula: X-Z (wherein X represents a peptide and Z represents a labeling substance) is also exemplified.

The protecting group (Y) is not particularly limited, and a known protecting group applicable to a peptide can be used. As such a protecting group, a benzyloxycarbonyl group, a tert-butoxycarbonyl group, a benzyl group, a benzoyl group, an acetyl group, and the like are exemplified.

The peptide (X) is not particularly limited as long as it is a peptide having a sequence recognized by an activated Limulus factor. As the peptide (X), Asp-Pro-Arg (DPR), Val-Pro-Arg (VPR), Leu-Thr-Arg (LTR), Met-Thr-Arg (MTR), Leu-Gly-Arg (LGR), Ile-Glu-Gly-Arg (IEGR) (SEQ ID NO: 1), Glu-Gly-Arg (EGR), and the like are exemplified.

The labeling substance (Z) is not particularly limited, and a known labeling substance applicable to a peptide can be used. Such a labeling substance may be a substance that is detected by ultraviolet light or visible light, or may be a fluorescent dye, or may be a substance that is detected by electrochemical measurement. As the labeling substance (Z), paranitroaniline (pNA), 7-methoxycoumarin-4-acetic acid (MCA), 2,4-dinitroaniline (DNP), a dansyl-type dye, a rhodamine-type dye, a cyanine-type dye, phenylenediamine (PDA), a derivative thereof, and the like are exemplified.

As the substrate, a preferred substrate can be appropriately selected and used according to the type of the Limulus factor to be detected. For example, from the viewpoint of substrate specificity, a substrate containing the peptide sequence of LGR or IEGR can be preferably used for the measurement of clotting enzyme, a substrate containing the peptide sequence of LTR or MTR can be preferably used for the measurement of factor B in an active form, a substrate containing the peptide sequence of VPR or DPR can be preferably used for the measurement of factor C in an active form, and a substrate containing the peptide sequence of EGR can be preferably used for the measurement of factor G in an active form.

It is preferred that the drug and the auxiliary agent do not substantially contain a microbial contaminant. This is because a mixed solution obtained by mixing the drug and the auxiliary agent can be administered to animals including humans as it is, or it can be used for preparation of a sample for measurement for a microbial contaminant test.

Here, the phrase "not substantially contain a microbial contaminant" means that the amount of a microbial contaminant to be detected from the drug and the auxiliary agent is less than the limit of quantification or less than the limit of detection.

### (2) Others

The drug and the auxiliary agent may contain a known material that is generally contained in a drug or an auxiliary agent. For example, when the drug and the auxiliary agent are each the below-mentioned lyophilizate, an inorganic component such as sodium chloride, an organic component such as a surfactant, or the like that is contained in an aqueous solvent in which the drug or the auxiliary agent is dissolved before lyophilization may be contained.

### 4. Form

The form of the drug and the auxiliary agent is not particularly limited, and may be a solid form or a liquid form (liquid), or may be a state where a liquid form (liquid) is frozen. In the below description, the drug in a liquid form is sometimes referred to as a drug solution, and the drug in a solid form is sometimes referred to as a solid drug. Further, the auxiliary agent in a liquid form is sometimes referred to as an auxiliary agent solution, and the auxiliary agent in a solid form is sometimes referred to as a solid auxiliary agent.

As the drug solution or the auxiliary agent solution, for example, a mixed liquid obtained by mixing (dissolving) the drug or the auxiliary agent in the aqueous solvent is exemplified. The aqueous solvent is as described above. The mixed liquid is appropriately prepared so that the mixed liquid shows the pH or the molar concentration of the buffer described above.

On the other hand, as the solid drug or the solid auxiliary agent, for example, a lyophilizate of the drug or the auxiliary agent, a lyophilizate of the above-mentioned mixed liquid, and a solid preparation containing the drug or the auxiliary agent are exemplified. Above all, the solid preparation containing the drug or the auxiliary agent enables stabilization of the form, and therefore is preferred. As the solid preparation, for example, a solid preparation containing the drug or the auxiliary agent and a molding agent, a capsule obtained by filling the drug or the auxiliary agent in a known capsule case or by encapsulation molding thereof with a capsule film, and the like are exemplified.

As the solid preparation containing the drug or the auxiliary agent and the molding agent, for example, a molded material alone obtained by molding a mixture of the drug or the auxiliary agent and the molding agent into a desired form such as a tablet or a powder, and other than this, a capsule obtained by filling the drug or the auxiliary agent or the molded material in a known capsule case or by encapsulation molding thereof with a capsule film can also be included. As the form of the solid preparation, a tablet, a pill, a capsule, a powder, a granule, and the like are exemplified, and above all, a tablet, a pill, and a capsule are preferred, and particularly a tablet is preferred. Note that the "molding agent" as used herein is a substance that is directly mixed with the drug or the auxiliary agent, and shall not include a material that is not mixed with the drug or the auxiliary agent such as a molding agent contained in the capsule case of the "capsule".

When the solid preparation contains a molding agent, the drug or the auxiliary agent and the molding agent exist dispersedly in one unit (one piece) of the solid preparation. When the auxiliary agent contained in the solid preparation contains two or more types of the above-mentioned auxiliary agent components, the solid preparation can be prepared as a formulation containing two or more types of the above-mentioned auxiliary agent components and the molding agent in one unit (one piece).

As the molding agent, a known material generally used for manufacture of a solid preparation can be used. As the molding agent, for example, an excipient, a binder, and the like are exemplified. Specifically, cellulose such as crystalline cellulose; starch; dextrin; dextran; sugar alcohols such as mannitol, erythritol, xylitol, maltose, maltitol, and sorbitol; saccharides such as lactose, white soft sugar, trehalose, and glucose; phosphates such as calcium phosphate and sodium phosphate; sodium chloride; calcium carbonate; kaolin; silicic acid, and the like can be exemplified. Among these materials, one type can be used singly or two or more types can be used in combination.

When the solid preparation contains a molding agent, the content of the molding agent contained in the solid preparation may be an amount capable of molding the drug or the auxiliary agent into a desired form, and can be appropriately set according to the form of the solid preparation, the content of the drug or the auxiliary agent in the solid preparation, or the like. Above all, the content of the molding agent is preferably an amount capable of forming the form of the solid preparation into a tablet. The content of the molding agent in the solid preparation in terms of mass concentration (w/w%) can be set to, for example, 1 w/w% or more, 5 w/w% or more, 10 w/w% or more, 25 w/w% or more, or 50 w/w% or more. Further, the content of the molding agent in terms of mass concentration (w/w%) can be set to, for example, 99.9 w/w% or less, 99 w/w% or less, 95 w/w% or less, 90 w/w% or less, 80 w/w% or less, 70 w/w% or less, or 60 w/w% or less.

It is preferred that at least one of the drug and the auxiliary agent is in a solid form, and it is more preferred that both the drug and the auxiliary agent are in a solid form. This is because it becomes easy to allow the drug and the auxiliary agent to exist separately in the closed space.

When the drug is a solid drug, the solid drug preferably does not substantially contain an aqueous solvent. This is because the active ingredient of the drug can be stably stored as compared with the case where an aqueous solvent is contained. The phrase "not substantially contain an aqueous solvent" means that the moisture content (w/w%) in the solid drug is, for example, within a range of 20 w/w% to 0 w/w%, preferably within a range of 10 w/w% to 0 w/w%, more preferably within a range of 5 w/w% to 0 w/w%, further more preferably within a range of 2 w/w% to 0 w/w%, and particularly preferably within a range of 1 w/w% to 0 w/w%.

When the drug is a solid drug, the solid drug may be a lyophilizate or a solid preparation, however, when the drug is retained on the closing member, the solid drug is preferably a solid preparation. This is because the retention in the closing member becomes easy. On the other hand, when the drug exists in the container body (other than the closing member), the solid drug is preferably a lyophilizate. This is because inactivation of the drug can be prevented.

Similarly, when the auxiliary agent is a solid auxiliary agent, the solid auxiliary agent may be a lyophilizate or a solid preparation, however, when the auxiliary agent is retained on the closing member, the solid auxiliary agent is preferably a solid preparation. This is because the retention in the closing member becomes easy. On the other hand, when the auxiliary agent exists in the container body (other than the closing member), the solid auxiliary agent may be a solid preparation or a lyophilizate.

When the auxiliary agent is a solid auxiliary agent containing a buffer as an auxiliary agent component, the amount of the buffer contained in the solid auxiliary agent is preferably such an amount that the molar concentration of the buffer when it is mixed with a predetermined aqueous solvent falls within the range described in the above-mentioned section "2. Auxiliary Agent". The content of the buffer in the solid preparation in terms of mass concentration (w/w%) can be set to 0.01 w/w% or more, 0.05 w/w% or more, 0.1 w/w% or more, or 1 w/w% or more. Further, the content in terms of mass concentration (w/w%) can be set to 50 w/w% or less, 20 w/w% or less, 10 w/w% or less, 5 w/w% or less, or 1 w/w% or less.

### B. Closing Member

The closing member in the present invention is a member that closes the opening portion of the container body. Here, the phrase "closes the opening portion of the container body" means that the opening portion of the container body is closed so as to prevent contamination with another material (solid) through the opening portion into the container body by the closing member. In a state where the opening portion of the container body is closed, the closing member may or may not seal the container body, but preferably seals the container body. The term "seal" means that the drug and the auxiliary agent in the container body do not leak out, and gas permeation does not substantially occur.

The closing member may be connected to and integrated with the container body, or may be a separate body separated from the container body.

The closing member is not particularly limited as long as it can close the opening portion of the container body, and can be appropriately selected according to the type of the container body and the form of the opening portion. Examples of the closing member include a lid, a pot lid, a cap, a plug, and a composite thereof. Examples of the cap include a screw cap and a hinge cap. Further, examples of the plug include a plug-in-type plug and a covering-type plug.

The closing member is not particularly limited as long as it has a shape capable of closing the opening portion of the container body, and for example, may have a planar face at the opening portion side of the closing member, and as shown in FIGS. 4(a) to 4(e), it can have a top plate portion 2a and a projection portion T located at a position overlapping with the opening portion O on a face of the top plate portion 2a at the closing member 1 side in plan view. This is because when the opening portion of the container body is closed by the closing member, the projection portion is inserted into the opening portion and located in the container body, and can retain the solid preparation on the surface of the projection portion (FIG. 4(a)), or can retain the solid preparation between the projection portion and the inner wall of the container body (FIG. 4 (b)). As illustrated in FIG. 4(c), when the closing member 2 has two or more projection portions T, the solid preparation may be pinched by the two or more projection portions T. In addition, as illustrated in FIG. 4(d), the projection portion T may have a protruding portion t protruding in a direction crossing the insertion direction into the opening portion. This is because if the solid preparation is a tablet with a hole, the solid preparation can be hung and retained by passing the protruding portion through the hole of the solid preparation. Further, as illustrated in FIG. 4(e), the projection portion T may have a recess portion P on a side face or a face crossing the fit-in direction. This is because the solid preparation can be retained on the recess portion.

Further, as illustrated in FIG. 5, the closing member may have the top plate portion 2a, and the recess portion P located at a position overlapping with the opening portion O on a face of the top plate portion 2a at the closing member 1 side in plain view. This is because the solid preparation can be retained on the recess portion. It is preferred that the recess portion exists on a face crossing the center axis of the opening of the opening portion of the container body.

It is preferred that the closing member can be fitted to the opening portion of the container body. This is because by fitting the closing member inside or outside the opening portion of the container body, the drug and the auxiliary agent can be stored in a sealed state. Among the closing members, a closing member that can be fitted to the opening portion of the container body is sometimes particularly referred to as a sealing member. The condition in which the closing member can be fitted to the opening portion of the container body may be a condition in which the closing member can be fitted into the opening portion of the container body or can be fitted outside the opening portion of the container body.

As the closing member that can be fitted into the opening portion of the container body, for example, a closing member having a plug portion that is fitted into the opening portion of the container body is exemplified. Further, as the closing member that can be fitted outside the opening portion of the container body, for example, a closing member having a top plate portion and a rib that is formed on a face of the top plate portion at the closing member 1 side and is fitted to the peripheral edge of the opening of the opening portion of the container body, a closing member having a top plate portion and a tubular surrounding wall portion that hangs down from the peripheral edge of the top plate portion and is fitted outside the opening portion, and the like are exemplified.

Above all, the closing member preferably has a plug portion that is fitted into the opening portion of the container body. The closing member 2 in FIGS. 1(a) and 1(b) and FIGS. 2(a) and 2(b) has a plug portion (2b in FIGS. 1 and 2) that is fitted into the opening portion O of the container body 1.

When the closing member includes a plug portion, as shown in FIGS. 2(a) and 2(b), the closing member 2 may have the top plate portion 2a and the plug portion 2b on a face of the top plate portion 2a at the closing member 2 side, or as shown in FIG. 3, the entire closing member 2 may be the plug portion 2b.

The plan-view shape of the plug portion is not particularly limited as long as it is a shape capable of being fitted into the opening portion of the container body, and for example, a circular shape, a quadrangular shape, or the like can be adopted. In FIG. 2, a plug portion whose plan-view shape is a circular shape is shown. Further, the longitudinal cross-sectional shape in the fit-in direction of the plug portion is not particularly limited, and for example, a triangular shape, a quadrangular shape, a tapered shape, and the like are exemplified. In addition, the tip of the plug portion may have a sharp shape or a flat shape, or may have a shape with a curved face.

The height of the plug portion (the vertical length in the fit-in direction) is appropriately designed so that the plug portion can be fitted into the opening portion of the container body so as to enable sealing. Above all, the height is preferably a height with a temporarily plugging portion so as to enable temporary fitting when the plug portion is fitted into the opening portion.

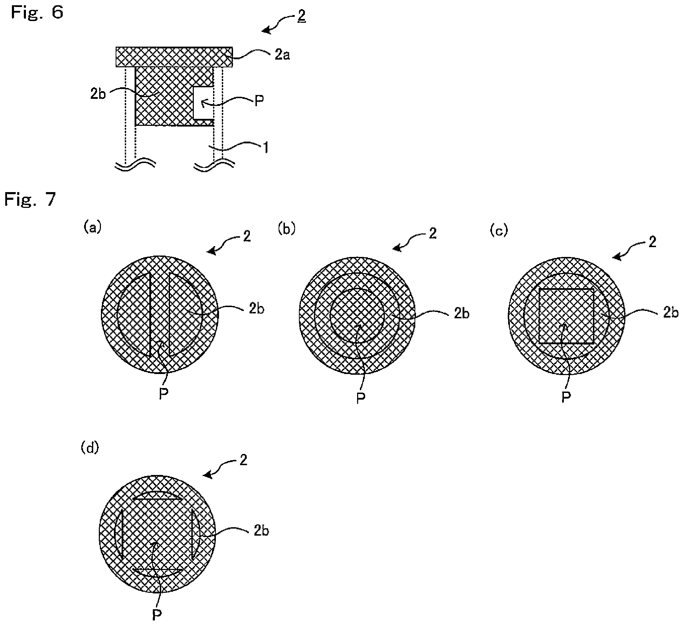

The plug portion can have the recess portion for fitting the solid preparation containing the drug or the auxiliary agent therein. The position of the recess portion P is not particularly limited, and for example, as illustrated in FIG. 6, it can be included on a side face of the plug portion 2b. In that case, the solid preparation is fitted to the recess portion, and the solid preparation can be retained between the plug portion and the inner wall of the container body. In addition, the plug portion can have the recess portion P on a face crossing the fit-in direction Y as shown in FIGS. 2(a) and 2(b). When the plug portion has the recess portion on a face crossing the fit-in direction, the recess portion is formed so that the fit-in direction becomes equal to the depth direction of the recess portion. By configuring the plug portion to have the above structure, for example, the solid preparation containing the drug or the auxiliary agent can be fitted to the recess portion and retained therein.

The recess portion preferably has a shape capable of retaining the solid preparation. When the plug portion has the recess portion P on a face crossing the fit-in direction Y, the plan-view shape of the recess portion is not particularly limited, but for example, a line shape (FIG. 7(a)), a circular shape (FIG. 7(b)), a polygonal shape such as a triangular shape or a quadrangular shape (FIGS. 7(c) and 7(d)), and the like are exemplified.

In the case where the plug portion has the recess portion on a face crossing the fit-in direction, it is preferred that the recess portion has an enough depth to expose a part through the opening portion of the container body when the plug portion is temporarily fitted to the opening portion of the container body. At that time, it is more preferred that the part of the recess portion penetrates the side face of the plug portion. This is because in a process for manufacture of the drug storage container of the present invention, when a liquid material exists in the container body (in the closed space) separately from the solid preparation that is retained on the closing member is lyophilized in a state where the opening portion of the container body is temporarily fitted with the closing member, the recess portion can function as a degassing channel for air in the container body, and as a result, the liquid material can be lyophilized with high efficiency.

As the shape of the recess portion that penetrates the side face of the plug portion, for example, a shape in which the plan-view shape of the recess portion is a line shape, and both ends of the recess portion in a line shape in the longitudinal direction penetrate the side face of the plug portion as shown in FIG. 7(a), a shape in which the plan-view shape of the recess portion is a quadrangular shape, and the four corners of the recess portion in the quadrangular shape penetrate the side face of the plug portion as shown in FIG. 7(d), and the like are exemplified.

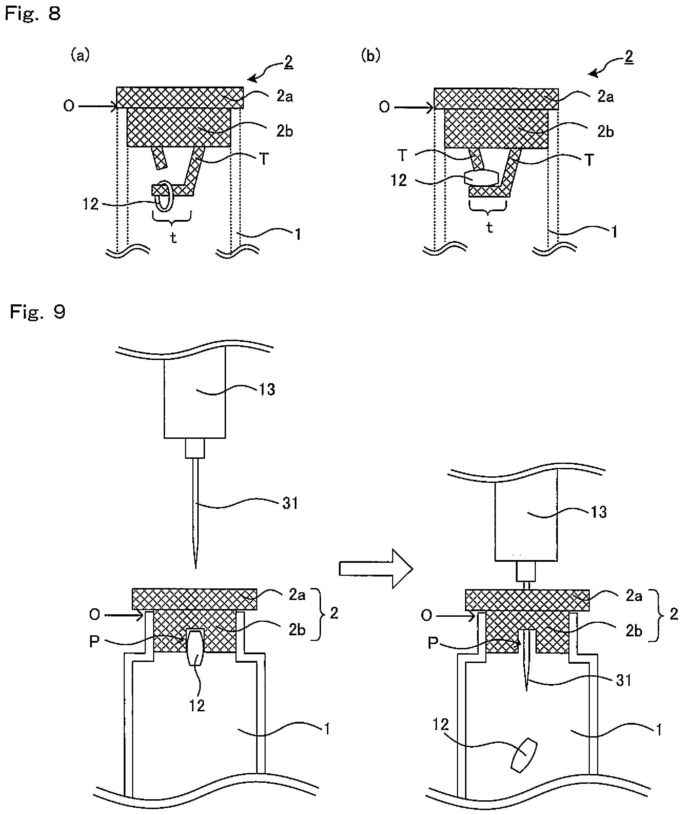

The plug portion can further have one or more projection portions T on the face crossing the fit-in direction Y as illustrated in FIG. 8(a). When it has two or more projection portions T, the solid preparation can be pinched by the two or more projection portions T. In addition, the projection portion may have a protruding portion t protruding in a direction crossing the fit-in direction at an end on the opposite side to the plug portion. If the solid preparation is a tablet with a hole, the solid preparation 12 can be hung and retained by passing the protruding portion t through the hole of the solid preparation 12 as illustrated in FIG. 8(a). FIG. 8(b) shows an example in which the closing member 2 has two projection portions T on a face of the plug portion 2b crossing the fit-in direction Y, and one of the projection portions T further has a protruding portion t, and the solid preparation 12 is retained by the two projection portions T.

A material of the closing member is not particularly limited as long as it is a material capable of closing the opening portion of the container body and can be appropriately selected according to the shape of the closing member, the shape of the opening portion of the container body, or the like. Examples of the material of the closing member include a metal such as aluminum, a resin, and a rubber. Further, when the closing member has a plug portion, as a material of the plug portion, an elastic body such as a resin or a rubber is preferred. This is because a state where it is fitted into the opening portion of the container body can be maintained, and the air tightness of the inside of the closed space can be enhanced.

The closing member may be used in a state of a processed material as it is, or may be used in a state of being treated by coating or the like. This is because the solid preparation can be more reliably retained on the closing member or detached therefrom.

The closing member is preferably configured such that a hollow needle such as an injection needle can penetrate the closing member although it depends on the material thereof. This is because it becomes possible to put an aqueous solvent into the container body using a syringe or the like without opening or closing the closing member, so that contamination with an impurity into the container body due to opening or closing of the closing member can be prevented. In addition, this is because when the solid preparation is detachably retained on the closing member, above all, when the solid preparation is physically detachably retained on the closing member, the solid preparation can be detached from the closing member by pressing the solid preparation retained on the closing member with a hollow needle penetrating the closing member at the time of putting of the aqueous solvent.

When the closing member has the plug portion, by allowing a hollow needle to penetrate at a position overlapping with the recess portion included in the plug portion in plan view, for example, as shown in FIG. 9, by pressing the auxiliary agent 12 (the solid preparation containing the auxiliary agent 12 in FIG. 9) retained on the recess portion P of the plug portion 2b at the time of putting of an aqueous solvent, the auxiliary agent 12 can be detached from the recess portion P, and therefore can be efficiently mixed in the aqueous solvent together with the drug (not shown) that exists separately in the container body 1.

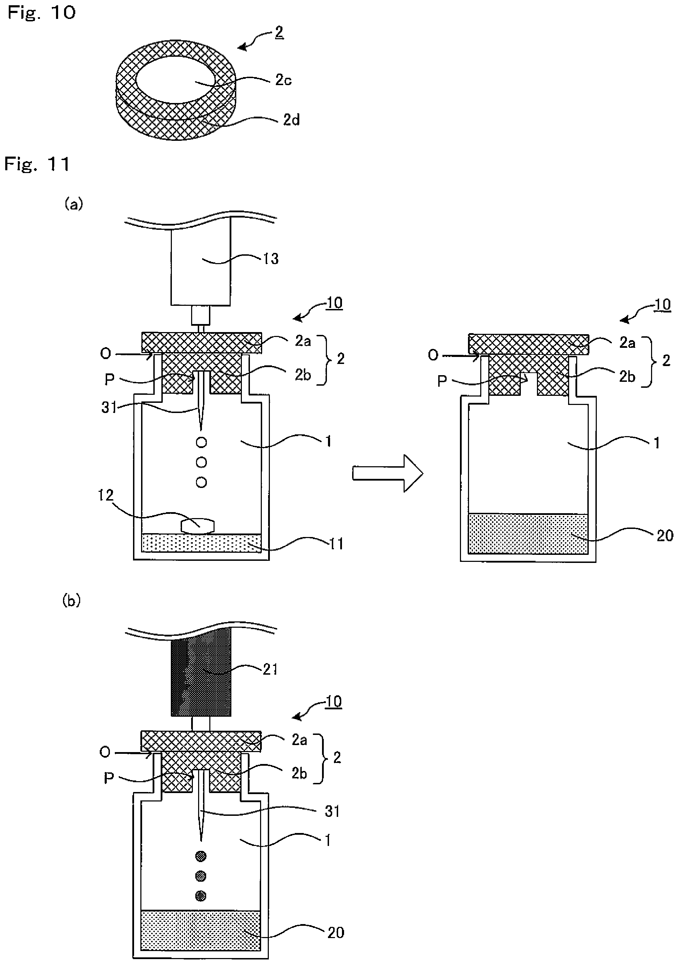

The closing member may have a region in which a hollow needle can penetrate (puncturable region) in a portion. This is because even if the closing member is made of a material that is difficult to be pierced by a hollow needle, the hollow needle can penetrate the closing member in the puncturable region, and therefore, it becomes possible to put an aqueous solvent without opening or closing the closing member. In addition, this is because when the solid preparation is detachably retained on the closing member, above all, when the solid preparation is physically detachably retained on the closing member, the solid preparation can be detached from the closing member by pressing the solid preparation retained on the closing member with a hollow needle penetrating the puncturable region at the time of putting of an aqueous solvent. As the closing member having a puncturable region, for example, as shown in FIG. 10, a structure having a puncturable region 2c formed from a rubber, a resin, or the like, and a casing portion 2d formed from a metal and surrounding the puncturable region 2c is exemplified.

When the closing member has a plug portion, it is preferred to have the puncturable region at a position overlapping with a recess portion included in the plug portion in plan view. This is because the solid preparation can be detached from the closing member by pressing the solid preparation retained on the recess portion with a hollow needle penetrating the puncturable region at the time of putting an aqueous solvent.

### C. Container Body

The container body in the present invention has an opening portion at one end.

The container body is not particularly limited as long as it has a shape having at least one opening portion, and may have a shape having two or more opening portions . In addition, the container body is not particularly limited as long as it has a shape having an opening portion at least at one end, and for example, may have a shape having another opening portion (second opening portion) at a position different from that of the opening portion at one end (first opening portion).

As a specific shape of the container body, for example, a tubular shape, a cup shape, a bag shape, and the like are exemplified. Specific examples of the container body having such a shape include a vial, a syringe, and an infusion bag.

When the container body has two or more opening portions, each opening portion is closed by the closing member. When the container body has two or more opening portions, the drug or the auxiliary agent only needs to be retained on one or two or more closing members among a plurality of closing members that close the opening portions. When the container body has two or more closing members, the same drug or auxiliary agent may be retained on each of the two or more closing members, or drugs or auxiliary agents that are different for each closing member may be retained therein. In addition, when the container body has two or more closing members, the two or more closing members may have the same form or different forms.

The material of the container body is not particularly limited, and for example, a metal, a resin, and a glass can be exemplified, however, among these, a glass is preferred from the viewpoint of a high gas barrier property.

The container body may be transparent, semi-transparent, or opaque, but is preferably transparent or semi-transparent from the viewpoint of ease of visibility of the contents of the container body.

The inside of the container body may be pressurized or depressurized. The degree of pressurization or depressurization can be appropriately adjusted according to the type or amount of the drug or the auxiliary agent existing in the container body, the purpose of use of the container body, or the like. Further, with respect to the container body, the gas in the container body may be air, but may be replaced with nitrogen. This is because deterioration of the drug or the auxiliary agent in the container body can be prevented.

### D. Others

In the drug storage container of the present invention, in the closed space formed by closing the opening of the container body by the closing member, the drug and the auxiliary agent exist separately. In the drug storage container of the present invention, it is preferred that the drug and the auxiliary agent are enclosed in the container body that is sealed by the closing member (that is, in the sealed space). Note that the term "sealed" is as already described.

The mode in which the drug and the auxiliary agent exist separately is not particularly limited, however, for example, a mode in which the drug and the auxiliary agent are retained separately in the container body, a mode in which one of the drug and the auxiliary agent is detachably retained on the closing member, and the like are exemplified. As the mode in which the drug and the auxiliary agent are retained separately in the container body, for example, one of the drug and the auxiliary agent is retained on the wall face in the container body, and the other may be retained at a different position on the wall face in the container body or may not be retained on the wall face. In addition, as the mode in which one of the drug and the auxiliary agent is detachably retained on the closing member, for example, a mode in which one of the drug and the auxiliary agent is detachably retained on the closing member, and the other exists in the container body other than the closing member can be adopted. The term "detachably" will be described later.

The other substance of the drug and the auxiliary agent, which is not retained on the closing member or on the wall face in the container body, may exist in a liquid form (liquid) or may exist in a solid form, or may exist in a state where a liquid form (liquid) is frozen. It is preferred that moisture does not exist in the closed space from the viewpoint of long-term storage, and therefore, it is preferred that the other substance is in a solid form. Further, when the container body is used in a mode in which it is stored in a frozen state, the other substance may exist in a liquid form, or may exist in a solid form. The other substance in a solid form may be retained at a desired position in the container body, or may not be retained.

Above all, the mode in which the drug or the auxiliary agent is detachably retained on the closing member is preferred. This is because by adopting such a mode, it becomes easy to allow the drug and the auxiliary agent to exist separately in the closed space, and by detaching the drug or the auxiliary agent retained on the closing member, preparation of the mixed solution can be easily carried out.

Here, the condition in which it is "detachable" may be a condition in which the substance retained on the closing member is physically detachable by receiving a force or the like, or may be a condition in which the substance retained on the closing member is chemically detachable by causing a reaction such as dissolution by contact with the other substance. As the condition in which it is physically detachable, for example, a case where a hollow needle such as an injection needle is allowed to penetrate the closing member so that the substance such as a tablet can be detached by the pressing force of the hollow needle, a case where the drug storage container is centrifuged or the drug storage container is shaken by flicking the wrist so that the substance such as a tablet can be detached, and the like are exemplified. Further, as the condition in which it is chemically detachable, for example, a case where as a result of causing a reaction such as dissolution by contact with the aqueous solvent in the container body by tumbling stirring or vertically shaking the drug storage container, the substance such as a tablet can be detached, and the like are exemplified.

The drug or the auxiliary agent detachably retained on the closing member is preferably in a solid form, and is particularly preferably a solid preparation. This is because by using the solid preparation, it becomes easy to retain the drug or the auxiliary agent on the closing member and to detach the drug or the auxiliary agent from the closing member. At that time, the form of the solid preparation is more preferably a tablet. Note that it is more preferred that between the drug and the auxiliary agent, the auxiliary agent is retained on the closing member.

When the opening portion of the container body is closed by the closing member, the drug or the auxiliary agent may be retained on a face located at the opening portion side of the faces of the closing member. The mode in which the drug or the auxiliary agent is retained on the closing member is not particularly limited as long as it is a mode enabling the detachment from the closing member, and can be appropriately selected according to the form of the drug or the auxiliary agent or the shape of the closing member.

As a mode in which the drug or the auxiliary agent is retained in the drug storage container with the opening portion closed by the closing member, for example, in one mode, the closing member has a projection portion inserted inside the opening portion of the container body, and the drug or the auxiliary agent may be retained by the projection portion of the closing member. Further, in one mode, the closing member has a recess portion in the container body (in the closed space), and the drug or the auxiliary agent may be retained on the recess portion of the closing member.

As a specific mode in which the drug or the auxiliary agent is retained by the projection portion or the recess portion, although not particularly limited to, for example, a mode in which the drug or the auxiliary agent is adhered to a face of the projection portion or the recess portion of the closing member through an adhesive substance, a mode in which a solid preparation containing the drug or the auxiliary agent is pinched by two or more projection portions included on the closing member, a mode in which a solid preparation containing the drug or the auxiliary agent is fitted to the recess portion included on the closing member, and the like are exemplified. At that time, the drug or the auxiliary agent preferably exists on a face crossing the center axis of the opening portion of the container body.

When the projection portion further has a protruding portion, as illustrated in FIG. 4(d), for example, a tablet with a hole of the auxiliary agent 12 can be retained by a protruding portion t. In addition, as illustrated in FIG. 8(b), for example, a solid preparation of the auxiliary agent 12 can be pinched between a protruding portion t of one projection portion T and the other projection portion T.

Further, the closing member has the recess portion in the projection portion, and the drug and the auxiliary agent may be retained on the recess portion. This is because on the closing member, a state where the solid preparation is retained can be easily maintained. The recess portion of the closing member preferably exists on a face crossing the center axis of the opening portion of the container body when closing the container body by the closing member.

Further, when the closing member has a plug portion that is fitted into the opening portion of the container body, the mode in which the drug or the auxiliary agent is retained in the drug storage container with the opening portion closed by the closing member is not particularly limited as long as it is a mode enabling the detachment from the closing member, and for example, the drug or the auxiliary agent may be retained on a face crossing the fit-in direction of the plug portion of the closing member, or may be retained on a side face of the plug portion. At that time, the drug or the auxiliary agent may be retained by being fitted on the recess portion located on the face crossing the fit-in direction of the plug portion or the side face thereof. Above all, it is preferred that the plug portion of the closing member has a recess portion on a face crossing the fit-in direction, and the drug or the auxiliary agent is detachably retained on the recess portion of the plug portion. This is because the detachment from the closing member becomes easy.

As a preferred mode as the mode in which the drug and the auxiliary agent exist separately, a mode in which the closing member has a plug portion that is fitted into the opening portion of the container body, the plug portion has a recess portion on a face crossing the fit-in direction, a lyophilizate of the drug exists on an inner wall or a bottom face in the container body, and a solid preparation containing the auxiliary agent (solid auxiliary agent) is detachably retained on the recess portion of the plug portion is exemplified. The mode is preferred from the viewpoint that contamination of the drug and the auxiliary agent or inactivation of the drug is prevented, and further, the detachment of the solid preparation containing the auxiliary agent (solid auxiliary agent) becomes easy.

### E. Mixed Solution

The mixed solution obtained according to the present invention can be, for example, formed into a reagent solution for a microbial contaminant test (reagent solution), a sample for measurement, or a medicine such as a biological drug, or the like according to the types of a drug and an auxiliary agent and an aqueous solvent to be put into the container body.

The mixed solution obtained according to the present invention can be prepared by performing an operation of allowing the drug and the auxiliary agent to coexist in an aqueous solvent in the container body (in the closed space). Here, the term "coexist" means that the subject materials are in a state of being able to come into contact with each other. A method for allowing the drug and the auxiliary agent to coexist in an aqueous solvent is not particularly limited, however, for example, when a drug solution is used, a method of performing an operation in which a solid auxiliary agent retained on the closing member is detached and added into the drug solution in the container body is exemplified. Further, when a solid drug and a solid auxiliary agent are used, a method of performing an operation in which an aqueous solvent to be mixed with the solid drug and the solid auxiliary agent is put directly or indirectly through the closing member through the opening portion into the container body is exemplified. The aqueous solvent is the same as the contents described in the section "A. Drug and Auxiliary Agent 2. Auxiliary Agent", and therefore, the description thereof is omitted here.

Further, the mixed solution obtained according to the present invention can also be formed into a sample for measurement containing a drug and an auxiliary agent and an analyte by further putting the analyte into a reagent solution prepared by mixing the drug and the auxiliary agent.

### F. Use and Usage

The drug storage container of the present invention can be used for preparation of a mixed solution containing a drug and an auxiliary agent. The use and usage of the drug storage container of the present invention will be described.

### 1. Use for Microbial Contaminant Test

The drug storage container of the present invention can be used for preparation of a reagent solution for a microbial contaminant test (reagent solution) or a sample for measurement. In the drug storage container of the present invention, a sample for measurement can be prepared by, for example, preparing a reagent solution, and thereafter putting an analyte into the drug storage container containing the reagent solution. Further, in the drug storage container of the present invention, a sample for measurement can also be directly prepared by, for example, putting only an analyte into the drug storage container without performing an operation of preparing a reagent solution. A test for a microbial contaminant can be carried out using the sample for measurement. That is, the drug storage container of the present invention can also be used as a container for a microbial contaminant test.

FIG. 11 is a schematic view showing an example of a usage of the drug storage container of the present invention, and shows an example of a method for preparation of a reagent solution for a microbial contaminant test and a test method for a microbial contaminant using the reagent solution. First, as shown in FIG. 11(a), an aqueous solvent 13 is put into a container body 1 of a drug storage container 10 of the present invention. In FIG. 11(a), a hollow needle 31 such as an injection needle is allowed to penetrate a closing member 2 so as to put the aqueous solvent 13 into the container body 1 from the hollow needle 31. A drug 11 and an auxiliary agent 12 in the container body 1 are mixed with the aqueous solvent 13, whereby a mixed solution (reagent solution) 20 is prepared. In the mixing of the drug 11, the auxiliary agent 12, and the aqueous solvent 13, it is preferred to perform shaking or stirring using a stirrer or the like.

Subsequently, as shown in FIG. 11(b), an analyte 21 is put into the container body 1 of the drug storage container 10 and mixed with the mixed solution (reagent solution) 20, whereby a sample for measurement containing the drug, the auxiliary agent, and the analyte is prepared. The amount of a microbial contaminant in the analyte can be calculated by, for example, measuring a turbidity or a color developed by a chromogenic group or fluorescence caused by a fluorescent group using an optical detector. Specifically, for example, the amount of coagulin generated from coagulogen by cleavage with an activated Limulus factor or the amount of a chromogenic group released from a substrate for detection is measured based on an absorbance or a transmittance, or the amount of a fluorescent group released from a substrate for detection is measured based on a fluorescence intensity, whereby the presence or absence of a microbial contaminant can be determined or the amount of a microbial contaminant can be calculated.

In the example shown in FIG. 11, the sample for measurement is prepared by putting an analyte after preparation of a reagent solution containing the drug 11 and the auxiliary agent 12, however, by putting an analyte in a liquid form without putting the aqueous solvent 13, it is also possible to directly prepare the sample for measurement without performing preparation of the reagent solution. The mode in which the preparation of the sample for measurement is completed by putting only the analyte in a liquid form can be used as a preferred mode for use in the microbial contaminant test in the present invention. This is because measurement of a microbial contaminant can be simply and reliably carried out.

### 2. Use for Preparation of Medicine

The drug storage container of the present invention can be used in preparation for a medicine such as a biological drug. Further, the drug storage container of the present invention can also be used as an injector such as an enema or a syringe by, for example, attaching a nozzle or an injection needle to one end of the drug storage container after preparation of a medicine such as a biological drug. When the drug storage container is used as a syringe, it is preferred to use it by attaching a plunger rod or the like to the other end.

### 3. Others

The drug storage container of the present invention may be provided alone according to the use or may be provided as a kit including the same. The kit can include another article as a constituent component according to the use thereof. For example, when the use is use for a microbial contaminant test, the kit may include, other than the drug storage container of the present invention, distilled water, an injection needle, a syringe, a reference standard for a microbial contaminant, a package insert describing the information of the product, and the like as constituent components.

### II. Closing Member

The closing member of the present invention is for use in the above-mentioned drug storage container and configured to detachably retain the solid preparation containing the drug or the auxiliary agent.

According to the closing member of the present invention, when the closing member of the present invention is used in the drug storage container, even if another substance, which is contraindicated or not preferred for premixing, exists in the container body, it can be allowed to exist individually separately in the container body at a stage before preparation of a mixed solution. Further, the closing member detachably retains the solid preparation, and therefore, by detaching the solid preparation and putting an aqueous solvent as needed, a mixed solution with another substance existing in the container body can be prepared before use, and a mixed solution containing a drug or an auxiliary agent having poor storage stability in an aqueous solvent can be simply prepared.

The closing member of the present invention can have, for example, the structure described in the above section "I. Drug Storage Container B. Closing Member". The closing member of the present invention is used as a member for closing an opening portion of a container body having the opening portion at one end. In the present invention, for example, a solid preparation is detachably retained on the closing member. The details of the closing member of the present invention are described in the above section "I. Drug Storage Container B. Closing Member", and therefore, the description thereof is omitted here.

In the present invention, the solid preparation contains a drug or an auxiliary agent. The details of the drug and the auxiliary agent, and the form of the solid preparation are described in the above section "I. Drug Storage Container A. Drug and Auxiliary Agent", and therefore, the description thereof is omitted here.

Further, the solid preparation is a solid auxiliary agent containing a buffer as an auxiliary agent component, and is preferably a solid preparation containing the buffer in such an amount that the pH becomes neutral when it is mixed with a predetermined aqueous solvent. The details of the buffer in the solid preparation such as the reason for this, the specific range of pH, and the amount of the buffer are described in the above section "I. Drug Storage Container A. Drug and Auxiliary Agent", and therefore, the description thereof is omitted here.

The mode of retaining the solid preparation in the present invention is described in the above section "I. Drug Storage Container D. Others", and therefore, the description thereof is omitted here.

### III. Method for Manufacture of Drug Storage Container

The method for manufacture of a drug storage container of the present invention is a method for manufacture of the above-mentioned drug storage container, and the following three embodiments can be exemplified. The method for manufacture of a drug storage container of the present invention will be described for each embodiment.

### A. First Embodiment

The method for manufacture of a drug storage container of this embodiment is roughly classified into the following first example and second example.

The first example of the method for manufacture of a drug storage container of this embodiment includes a putting step of putting the drug through the opening portion into the container body, and a closing step of closing the container body in a state where the auxiliary agent is retained on the closing member while placing a face of the closing member on which the auxiliary agent is retained at the opening portion side of the container body.

Further, the second example of the method for manufacture of a drug storage container of this embodiment includes a putting step of putting the auxiliary agent through the opening portion into the container body, and a closing step of closing the container body in a state where the drug is retained on the closing member while placing a face of the closing member on which the drug is retained at the opening portion side of the container body.

According to the first embodiment of the method for manufacture of a drug storage container of the present invention, a drug storage container in which a drug and an auxiliary agent exist separately in the container body with the opening portion of the container body closed by the closing member (in a closed space) can be easily obtained.

Hereinafter, the respective steps of the method for manufacture of a drug storage container of this embodiment will be described.

### 1. Putting step

The putting step in this embodiment is a step of putting the drug through the opening portion into the container body (first example) or putting the auxiliary agent through the opening portion into the container body (second example).

The container body used in this step is described in the section "I. Drug Storage Container C. Container Body", and therefore, the description thereof is omitted here.

The drug or the auxiliary agent to be put into the container body in this step may be in a solid form or a liquid form. The drug in the first example of this step is preferably a solid drug, and is particularly preferably a lyophilizate. Further, the auxiliary agent in the second example of this step may be a solid auxiliary agent or an auxiliary agent solution, but is preferably a solid auxiliary agent. The reason for this is as already described.

The details of the drug and the auxiliary agent used in this step, and the form thereof, and the like are described in the section "I. Drug Storage Container", and therefore, the description thereof is omitted here.

In this step, a method for putting the drug or the auxiliary agent into the container body is not particularly limited, and can be appropriately selected according to the respective conditions such as the form of the drug or the auxiliary agent. For example, in the case of a drug solution or an auxiliary agent solution, an injection method using such as a syringe, or the like are exemplified. In addition, for example, in the case of a solid drug or a solid auxiliary agent, a throw-in method, or the like are exemplified.

### 2. Closing Step

The closing step in this embodiment is a step of closing the container body in a state where the auxiliary agent is retained on the closing member while placing a face of the closing member on which the auxiliary agent is retained at the opening portion side of the container body (first example) or closing the container body in a state where the drug is retained on the closing member while placing a face of the closing member on which the drug is retained at the opening portion side of the container body (second example).

The closing member used in this step is described in the section "I. Drug Storage Container B. Closing Member", and therefore, the description thereof is omitted here.

The auxiliary agent or the drug retained on the closing member may be in a form capable of existing separately from the drug or the auxiliary agent to be put into the container body, and is preferably in a solid form, and is more preferably a solid preparation containing the drug or the auxiliary agent, further more preferably a solid preparation containing the drug or the auxiliary agent and a molding agent, and particularly preferably a tablet containing the auxiliary agent and a molding agent. The reason for this and a specific method for retaining the auxiliary agent or the drug on the closing member are described in the section "I. Drug Storage Container", and therefore, the description thereof is omitted here.

The drug, the auxiliary agent, and the solid preparation containing the drug or the auxiliary agent used in this step are described in the section "I. Drug Storage Container", and therefore, the description thereof is omitted here.

In this step, the opening portion of the container body only needs to be closed by the closing member, and the container body may be sealed according to the shape of the closing member.

### 3. Another Step

In this embodiment, in addition to the putting step and the closing step, another step may be further included. For example, in this embodiment, a sealing step of sealing the container body may be further included after the closing step. This is because by the sealing step, the air tightness of the drug storage container can be enhanced, and contamination with an impurity or the like in the container body can be prevented. The sealing step may be, for example, a step of sealing the container body by sealing a region where the opening portion of the container body and the closing member come into contact with each other.

### B. Second Embodiment

The method for manufacture of a drug storage container of this embodiment is roughly classified into the following first example and second example.

The first example of the method for manufacture of a drug storage container of this embodiment includes a putting step of putting the drug in a liquid form through the opening portion into the container body, a closing step of closing the container body in a state where the auxiliary agent is retained on the closing member while placing a face of the closing member on which the auxiliary agent is retained at the opening portion side of the container body, and a drying step of lyophilizing the drug in a liquid form in the container body so that the drug is solidified.

Further, the second example of the method for manufacture of a drug storage container of this embodiment includes a putting step of putting the auxiliary agent in a liquid form through the opening portion into the container body, a closing step of closing the container body in a state where the drug is retained on the closing member while placing a face of the closing member on which the drug is retained at the opening portion side of the container body, and a drying step of lyophilizing the auxiliary agent in a liquid form in the container body so that the auxiliary agent is solidified.

According to the second embodiment of the method for manufacture of a drug storage container of the present invention, contamination of the auxiliary agent and the drug can be reliably prevented by lyophilizing the auxiliary agent or the drug in a liquid form in the container body so that the auxiliary agent or the drug is solidified in a state where the opening portion of the container body is closed by the closing member in which the drug or the auxiliary agent is retained. Accordingly, a drug storage container in which a drug and an auxiliary agent exist separately can be easily obtained. Further, by performing lyophilization by the above method, deterioration of the storage stability of the drug before preparing a mixed solution can be prevented.

Hereinafter, the respective steps of the method for manufacture of a drug storage container of this embodiment will be described.

### 1. Putting step

The putting step in this embodiment is a step of putting the drug in a liquid form through the opening portion into the container body (first example) or putting the auxiliary agent in a liquid form through the opening portion into the container body (second example).

The details of this step can be the same as those of the putting step in the above "A. First Embodiment" except that the drug is a drug solution or the auxiliary agent is an auxiliary agent solution.

### 2. Closing Step

The closing step in this embodiment is a step of closing the container body in a state where the auxiliary agent is retained on the closing member while placing a face of the closing member on which the auxiliary agent is retained at the opening portion side of the container body (first example) or closing the container body in a state where the drug is retained on the closing member while placing a face of the closing member on which the drug is retained at the opening portion side of the container body (second example).

The details of this step can be the same as those of the closing step in the above "A. First Embodiment". Note that in this embodiment, it is necessary to remove air from the inside of the container body in the below-mentioned drying step, and therefore, it is preferred that the container body is not sealed by the closing member.

### 3. Drying Step

The drying step in this embodiment is a step of lyophilizing the drug in a liquid form in the container body so that the drug is solidified (first example) or lyophilizing the auxiliary agent in a liquid form in the container body so that the auxiliary agent is solidified (second example).

In this step, a method for lyophilizing the drug solution or the auxiliary agent solution in the container body is not particularly limited, and can be the same as a general lyophilization method. For example, after the drug solution or the auxiliary agent solution in the container body is frozen with liquid nitrogen or the like, the aqueous solvent is vaporized using a lyophilizer under reduced pressure without heating, whereby the drug or the auxiliary agent can be dried and solidified.

The lyophilization conditions and the like may be conditions capable of sufficiently vaporizing the aqueous solvent contained in the drug solution or the auxiliary agent solution, and can be appropriately set according to the type of the aqueous solvent.

### 4. Another Step

In this embodiment, in addition to the putting step, the closing step, and the drying step, another step may be further included. For example, in this embodiment, a sealing step of sealing the container body may be further included after the drying step. The details of the sealing step can be the same as those described in the above section "A. First Embodiment

### 4. Another Step".

### C. Third Embodiment

The method for manufacture of a drug storage container of this embodiment is roughly classified into the following first example and second example.

The first example of the method for manufacture of a drug storage container of this embodiment includes a putting step of putting the drug in a liquid form through the opening portion into the container body, a closing step of closing the container body by temporarily fitting the plug portion of the closing member to the opening portion of the container body in a state where the auxiliary agent is retained on the plug portion of the closing member, and a drying step of lyophilizing the drug in a liquid form in the container body so that the drug is solidified in a temporarily fitted state.

Further, the second example of the method for manufacture of a drug storage container of this embodiment includes a putting step of putting the auxiliary agent in a liquid form through the opening portion into the container body, a closing step of closing the container body by temporarily fitting the plug portion of the closing member to the opening portion of the container body in a state where the drug is retained on the plug portion of the closing member, and a drying step of lyophilizing the auxiliary agent in a liquid form in the container body so that the auxiliary agent is solidified in a temporarily fitted state.

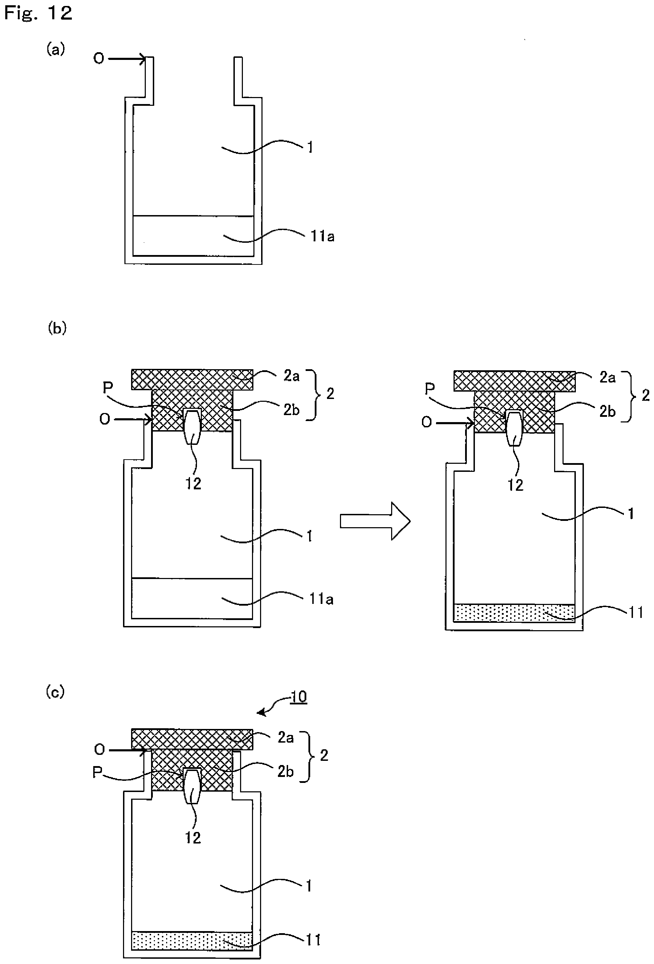

FIG. 12 is a process diagram showing an example of the method for manufacture of a drug storage container of this embodiment, and specifically illustrates the first example. First, a drug solution 11a is put from an opening portion O of a container body 1 (FIG. 12(a), putting step) . Subsequently, in a state where a solid preparation containing an auxiliary agent 12 is retained on a plug portion 2b of a closing member 2, the plug portion 2b of the closing member 2 is temporarily fitted to the opening portion O of the container body 1, whereby the container body 1 is closed (the left figure in FIG. 12(b), closing step). Subsequently, the drug solution 11a in the container body 1 in a temporarily fitted state is lyophilized so that the drug solution is solidified(the right figure in FIG. 12(b), drying step) . Thereafter, the plug portion 2b of the closing member 2 is fitted in the opening portion O of the container body 1, whereby the container body 1 is sealed (FIG. 12(c), sealing step) . A drug storage container 10 illustrated in FIG. 1 is obtained through, for example, the above operations shown in FIGS. 12(a) to 12(c). The reference signs not described in FIG. 12 are the same as the reference signs described in FIGS. 1 to 11.

According to the third embodiment of the method for manufacture of a drug storage container of the present invention, the drug or the auxiliary agent is retained on the plug portion of the closing member, and contamination of the auxiliary agent and the drug can be reliably prevented by lyophilizing the auxiliary agent or the drug in a liquid form in the container body so that the auxiliary agent or the drug is solidified in a state where the opening portion of the container body is temporarily fitted with the plug portion. Accordingly, a drug storage container in which a drug and an auxiliary agent exist separately can be easily obtained. In addition, by performing lyophilization by the above method, deterioration of the storage stability of the drug before preparing a mixed solution can be prevented. Further, since the opening portion of the container body is temporarily fitted with the plug portion, air in the container body can be easily removed from a gap at the temporary fitting position in the drying step, and therefore, lyophilization can be efficiently carried out, and also contamination with an impurity from the outside can be prevented.

Hereinafter, the respective steps of the method for manufacture of a drug storage container of this embodiment will be described.

### 1. Putting step

The putting step in this embodiment is a step of putting the drug in a liquid form through the opening portion into the container body (first example) or putting the auxiliary agent in a liquid form through the opening portion into the container body (second example).

The details of this step can be the same as those of the putting step in the above "B. Second Embodiment".

### 2. Closing Step

The closing step in this embodiment is a step of closing the container body by temporarily fitting the plug portion of the closing member to the opening portion of the container body in a state where the auxiliary agent is retained on the plug portion of the closing member (first example) or closing the container body by temporarily fitting the plug portion of the closing member to the opening portion of the container body in a state where the drug is retained on the plug portion of the closing member (second example).

In this step, the phrase "temporarily fitting the plug portion of the closing member to the opening portion of the container body" means that although a part of the plug portion closes the opening portion of the container body, the inside of the container body is not sealed, and air can go in and out of the container body.

The details of this step can be the same as those of the closing step in the above "B. Second Embodiment".

The closing member used in this step has the plug portion that is fitted into the opening portion of the container body. The plug portion preferably has the recess portion on a face crossing the fit-in direction, and particularly, the recess portion preferably has an enough depth to expose a part through the opening portion of the container body when the plug portion is temporarily fitted to the opening portion of the container body in the drying step. This is because when a drug solution or an auxiliary agent solution in the container body is lyophilized in a state of being temporarily fitted with the closing member in the below-mentioned drying step, a part of the recess portion included in the plug portion of the closing member is exposed through the opening portion of the container body so as to function as a degassing channel for air from the inside of the container body, and thus, lyophilization can be more efficiently carried out.

At that time, a method for retaining the drug or the auxiliary agent on the closing member is not particularly limited, but particularly, the drug or the auxiliary agent is preferably retained on the recess portion of the plug portion.

The details of the closing member, and the method for retaining the drug or the auxiliary agent on the closing member having the plug portion are described in the section "I. Drug Storage Container", and therefore, the description thereof is omitted here.

### 3. Drying Step

The drying step in this embodiment is a step of lyophilizing the drug in a liquid form in the container body so that the drug is solidified in a temporarily fitted state (first example) or lyophilizing the auxiliary agent in a liquid form in the container body so that the auxiliary agent is solidified in a temporarily fitted state (second example).

The details of this step can be the same as those of the drying step in the above "B. Second Embodiment".

### 4. Another Step

In this embodiment, in addition to the putting step, the closing step, and the drying step, another step may be further included. For example, in this embodiment, a sealing step of sealing the container body may be further included after the drying step. This is because by the sealing step, the air tightness of the drug storage container can be enhanced, and contamination with an impurity or the like in the container body can be prevented. The sealing step may be, for example, a step of sealing the container body by fitting the plug portion of the closing member in the opening portion of the container body.

### IV. Microbial Contaminant Test Method

The microbial contaminant test method of the present invention is a microbial contaminant test method for testing a microbial contaminant using the above-mentioned drug storage container, and includes a preparation step of preparing a sample that contains the drug and the auxiliary agent and an analyte and is subjected to measurement of a microbial contaminant (sample for measurement) by putting the analyte into the container body of the drug storage container, and a detection step of detecting the microbial contaminant in the sample.

According to the microbial contaminant test method of the present invention, by putting an analyte into the container body of the above-mentioned drug storage container, a sample for measurement containing a drug, an auxiliary agent, and an analyte can be prepared collectively in the container body. That is, according to the present invention, the sample for measurement can be easily prepared in one step without requiring a step of preparing and weighing an auxiliary agent solution, a step of preparing a reagent solution by putting the auxiliary agent solution into the container body containing a drug, or the like. Then, detection of a microbial contaminant can be carried out using the drug storage container in which preparation of the sample for measurement has been carried out.

In such a manner, according to the microbial contaminant test method of the present invention, preparation of a sample for measurement is easy, and determination of the presence or absence of a microbial contaminant in an analyte contained in the sample for measurement or measurement of the amount thereof can be simply and reliably carried out, and the degree of microbial contamination can be simply and reliably measured.

The effect brought about by the present invention will be described in more detail. In general, in the detection of a microbial contaminant, the preparation step requires at least an operation of preparing a reagent solution (mixed solution) by putting a required amount of a buffer solution that is weighed out into the container body in which a drug is enclosed, followed by stirring, and an operation of preparing a sample for measurement to be subjected to a detection step of detecting a microbial contaminant by putting the analyte into the reagent solution in the container body, followed by stirring. In such a manner, the conventional method had a problem that the preparation step is complicated. On the other hand, in the present invention, the drug storage container in which the drug and the auxiliary agent exist separately in advance in a sealed space is used, and therefore, only by an operation of putting an analyte into the drug storage container, a sample for measurement containing the drug and the auxiliary agent and the analyte can be prepared collectively in the drug storage container, and the production step can be simplified.

Hereinafter, the respective steps in the microbial contaminant test method of the present invention will be described.

### A. Preparation Step

The preparation step in the present invention is a step of preparing a sample for measurement by putting an analyte into the container body of the drug storage container.

The details of the drug storage container used in this step are described in the above section "I. Drug Storage Container", and therefore, the description thereof is omitted here.

In the drug storage container used in this step, a solid auxiliary agent is detachably retained on the closing member, and the solid auxiliary agent is preferably a solid auxiliary agent containing a buffer as an auxiliary agent component in such an amount that the pH becomes neutral when it is mixed with an analyte. The reason for this and the specific mode are described in the above section "I. Drug Storage Container", and therefore, the description thereof is omitted here.

The type of the analyte or the putting amount thereof in this step are not particularly limited as long as detection of a microbial contaminant in the analyte can be carried out, and can be appropriately set.

The analyte in the preparation step is put into the container body directly or indirectly through the closing member through the opening portion into the container body of the drug storage container. Examples of a method for putting the analyte into the container body of the drug storage container include a method for directly putting the analyte through the opening portion into the container body by detaching the closing member from the drug storage container, and a method for indirectly putting the analyte through a hollow needle without detaching the closing member through the opening portion into the container body by allowing the hollow needle to penetrate the closing member that closes the opening portion of the container body. When a solid preparation is retained on the closing member, the method for putting the analyte through a hollow needle by allowing the hollow needle to penetrate the closing member that closes the opening portion of the container body is preferred. This is because the solid preparation can be detached from the closing member at the same time as allowing the hollow needle to penetrate the closing member.

In this step, in the drug storage container, the analyte put in, and the drug and the auxiliary agent are mixed, whereby a sample for measurement is prepared.

This step is preferably carried out in the presence of an aqueous solvent. This is because the drug, the auxiliary agent, and the analyte can be mixed by being dissolved in the aqueous solvent. If at least one of the drug and the auxiliary agent is in a liquid form, the analyte may be in a solid form, or may be in a liquid form mixed in an aqueous solvent. On the other hand, if the drug and the auxiliary agent are in a solid form, the analyte is preferably an analyte solution (an analyte in a liquid form). This is because by an aqueous solvent in the analyte solution, the drug and the auxiliary agent are dissolved, and the analyte can be mixed with both. The aqueous solvent is described in the above section "I. Drug Storage Container A. Drug and Auxiliary Agent", and therefore, the description thereof is omitted here.

In this step, it is preferred to perform stirring using a test tube mixer, a shaker, a stirrer, or the like after putting the analyte so that the drug, the auxiliary agent, and the analyte are uniformly mixed in the container body.

### B. Detection Step

The detection step in the present invention is a step of detecting the microbial contaminant in the analyte. This step is specifically a step of detecting the microbial contaminant contained in the sample for measurement prepared in the preparation step as the microbial contaminant in the analyte.

The microbial contaminant is not particularly limited as long as it is a substance derived from a microorganism, but is preferably an endotoxin or (1→3)-β-D-glucan. This is because detection using a Limulus reagent can be carried out.

A method for detecting a microbial contaminant in an analyte is not particularly limited, and a method according to the type of a microbial contaminant can be used. When a Limulus reagent is used as the drug, examples of the detection method include an optical measurement method such as turbidimetry, a colorimetric method, or a fluorescence method, an electrochemical measurement method, and a gel-clot method. The details of such various detection methods are the same as various detection methods in a known Limulus test, and therefore, the description thereof is omitted here.

An optical detector to be used in the optical measurement method for a microbial contaminant can be appropriately selected according to the type of the optical measurement method or the like, and specifically, a spectrophotometer, a fluorophotometer such as a luminometer, and the like can be exemplified. As the electrochemical measurement method for a microbial contaminant, amperometry and voltammetry are exemplified. When the detection of a microbial contaminant is carried out by a gel-clot method, the presence or absence of a microbial containing can be determined by visually confirming the presence or absence of gel formation.

### V. Solid preparation for preparing Buffer Solution

The solid preparation for preparing a buffer solution of the present invention (hereinafter sometimes referred to as the solid preparation of the present invention) contains at least a buffer and a molding agent, in which the buffer is contained in such an amount that the pH becomes neutral when the solid preparation is mixed with a predetermined aqueous solvent.

According to the solid preparation of the present invention, the pH becomes neutral by mixing it with a predetermined aqueous solvent, and therefore, a buffer solution showing a predetermined pH can be easily prepared. Further, by using the solid preparation of the present invention when preparing a mixed solution containing a drug and an auxiliary agent, and a sample using the mixed solution, adjustment of the pH of the mixed solution or the sample can be easily carried out.

Specifically, when a sample for measurement to be used in a test for a microbial contaminant is prepared, by mixing the solid preparation of the present invention with a predetermined aqueous solvent, a buffer solution having a neutral pH is obtained without requiring dilution. By mixing the buffer solution as the auxiliary agent solution with a Limulus reagent (drug) in a liquid form or a solid form, a sample for measurement having a neutral pH can be obtained. In addition, by mixing a Limulus reagent and the solid preparation of the present invention with an aqueous solvent collectively, a sample for measurement having a neutral pH can be obtained without requiring preparation of a buffer solution.

The solid preparation of the present invention contains the buffer in such an amount that the pH becomes neutral when it is mixed with a predetermined aqueous solvent.

A specific range of the pH that is neutral is the same as the contents described in the section "I. Drug Storage Container", and therefore, the description thereof is omitted.

The content of the buffer in the solid preparation of the present invention in terms of mass concentration (w/w%) can be set to 0.01 w/w% or more, 0.05 w/w% or more, 0.1 w/w% or more, or 1 w/w% or more. Further, the content in terms of mass concentration (w/w%) can be set to 50 w/w% or less, 20 w/w% or less, 10 w/w% or less, 5 w/w% or less, or 1 w/w% or less.

Note that the mass concentration (w/w%) representing the content of the buffer in the solid preparation is the ratio (%) of the mass (g) of the buffer to the mass (g) of the entire solid preparation.

The content of the molding agent in the solid preparation of the present invention in terms of mass concentration (w/w%) can be set to, for example, 1 w/w% or more, 5 w/w% or more, 10 w/w% or more, 25 w/w% or more, or 50 w/w% or more. Further, the content of the molding agent in terms of mass concentration (w/w%) can be set to, for example, 99.9 w/w% or less, 99 w/w% or less, 95 w/w% or less, 90 w/w% or less, 80 w/w% or less, 70 w/w% or less, or 60 w/w% or less. Note that the mass concentration (w/w%) representing the content of the molding agent in the solid preparation is the ratio (%) of the mass (g) of the molding agent to the mass (g) of the entire solid preparation.

The final concentration of the buffer in the buffer solution obtained by mixing the solid preparation of the present invention with a predetermined aqueous solvent may be, for example, 1 mM or more, 5 mM or more, 10 mM or more, or 20 mM or more. The final concentration of the buffer may be for example, 1 M or less, 500 mM or less, 250 mM or less, or 100 mM or less. Therefore, as the range of the final concentration of the buffer, a range within a range of 1 mM to 1 M, within a range of 1 mM to 500 mM, within a range of 1 mM to 250 mM, within a range of 1 mM to 100 mM, within a range of 5 mM to 500 mM, within a range of 5 mM to 250 mM, within a range of 5 mM to 100 mM, within a range of 10 mM to 500 mM, within a range of 10 mM to 250 mM, within a range of 10 mM to 100 mM, within a range of 20 mM to 250 mM, within a range of 20 mM to 100 mM, or the like is exemplified.

The details of the buffer, the molding agent, and the aqueous solvent in the present invention are the same as the contents described in the section "I. Drug Storage Container", and therefore, the description thereof is omitted here.

It is preferred that the solid preparation of the present invention does not substantially contain a microbial contaminant. This is because the solid preparation of the present invention can be used for preparation of a mixed solution intended to be administered to animals including humans as it is or preparation of a sample for measurement for a microbial contaminant. The phrase "not substantially contain a microbial contaminant" means that the amount of a microbial contaminant (an endotoxin or (1→3)-β-D-glucan) to be detected from a buffer solution obtained by mixing the solid preparation of the present invention with a predetermined aqueous solvent is less than the limit of quantification or less than the limit of detection.

In the solid preparation of the present invention, at least a buffer and a molding agent are contained, and the buffer only needs to be contained in such an amount that the pH becomes neutral when the solid preparation of the present invention is mixed with a predetermined aqueous solvent, and another arbitrary component can be contained. Examples of the arbitrary component include materials of the drug or the auxiliary agent described in the above section "I. Drug Storage Container A. Drug and Auxiliary Agent".

Specifically, when the solid preparation of the present invention contains a predetermined amount of a buffer and a molding agent, and further contains at least one or more types selected from a solubilizing agent, an isotonizing agent, and a soothing agent, by mixing the solid preparation of the present invention with a predetermined aqueous solvent, a buffer solution having a neutral pH is obtained without requiring dilution. By mixing the buffer solution as the auxiliary agent solution with a protein formulation (drug) in a liquid form or a solid form, a biological drug having a neutral pH can be obtained. In addition, by directly mixing a protein formulation and the solid preparation of the present invention with an aqueous solvent collectively, a biological drug having a neutral pH can be obtained without requiring preparation of a buffer solution.

The form of the solid preparation of the present invention is not particularly limited, and for example, a tablet, a pill, a capsule, a powder, a granule, and the like are exemplified. Above all, a tablet, a pill, and a capsule are preferred, and particularly a tablet is preferred. This is because the tablet has a high strength as a solid preparation, and it is easy to retain it in the above-mentioned closing member of the present invention and maintain the retained state. When the solid preparation of the present invention is a tablet for preparation of a buffer solution containing at least a buffer and a molding agent, the buffer is contained in such an amount that the pH becomes neutral when one tablet is mixed with a predetermined aqueous solvent.

The solid preparation of the present invention can be manufactured using a general method for manufacture of a solid preparation. For example, in endotoxin-free (injection-grade) mannitol, 0.2 w/w% fine powder PBS is mixed by twin-screw kneading in an aseptic operation, and granules are prepared with a granulating device, followed by tableting, whereby the manufacture can be carried out. In the case of a finer PBS powder, the manufacture can be carried out by performing fine pulverization beforehand with a jet mill, and adding the resultant thereto similarly. Further, as a method for uniform granulation, a method in which PBS is dissolved in an endotoxin-free injection water, and the resulting solution is finely dispersed into mannitol with a spray-drying type kneading and granulating device, thereby manufacturing granules, or the like can be used. When the form is a tablet, a direct tableting method, a granule compression method, or the like can be used.

The solid preparation of the present invention can be adjusted to a neutral pH without requiring dilution when a buffer solution is prepared. The amount of the aqueous solvent required for preparation of a buffer solution using the solid preparation of the present invention can be set to such an amount that the pH becomes neutral without requiring dilution when it is mixed with the solid preparation, and for example, may be 0.1 mL or more or 0.2 mL or more although it depends on the amount of the buffer contained in the solid preparation. In addition, the amount of the aqueous solvent to be mixed with the solid preparation may be, for example, 1 L or less, 100 mL or less, 10 mL or less, 1 mL or less, or 0.5 mL or less. Therefore, as the range of the amount of the aqueous solvent to be mixed with the solid preparation, a range within a range of 0.1 mL to 1 L, within a range of 0.1 mL to 100 mL, within a range of 0.1 mL to 10 mL, within a range of 0.1 mL to 1 mL, within a range of 0.2 mL to 1 mL, within a range of 0.2 mL to 0.5 mL, or the like is exemplified. For example, in the case of a lysate reagent, the range of the amount can be set within a range of 0.1 mL to 10 mL, and is preferably within a range of 0.2 mL to 1 mL.

Note that the present disclosure is not limited to the above-mentioned embodiments. The above-mentioned embodiments are merely illustrative and matters having configurations which are substantially identical to the technical ideas described in the scope of the patent claims of the present invention and exhibiting the same operational effects fall within the technical scope of the present invention, whatsoever they may be.

Further, all publications, patent applications, and technical standards described herein are incorporated by reference herein to the same extent as if such individual publications, patent applications, and technical standards were specifically and individually indicated to be incorporated by reference.

### Reference Signs List

- 1:: container body
- 2:: sealing member
- 2b:: plug portion
- 10:: drug storage container
- 11:: solid protein formulation
- 12:: solid auxiliary agent
- 13:: aqueous solvent
- 20:: mixed solution
- 21:: analyte
- O:: opening portion
- P:: recess portion

## Claims

1. A drug storage container, comprising:
a container body having an opening portion at one end; and
a closing member that closes the opening portion of the container body, wherein
in the container body with the opening portion closed by the closing member, a drug and an auxiliary agent exist separately.

2. The drug storage container according to claim 1, wherein the drug is a medicine.

3. The drug storage container according to claim 1 or 2, wherein the auxiliary agent contains at least one or more that is selected from a solubilizing agent, an isotonizing agent, and a soothing agent as an auxiliary agent component.

4. The drug storage container according to claim 1, wherein the drug is a Limulus reagent.

5. The drug storage container according to any one of claims 1 to 4, wherein the auxiliary agent contains a buffer as an auxiliary agent component.

6. The drug storage container according to claim 5, wherein the auxiliary agent contains the buffer in such an amount that the pH becomes neutral when it is mixed with a predetermined aqueous solvent.

7. The drug storage container according to any one of claims 1 to 6, wherein the drug or the auxiliary agent is detachably retained on the closing member.

8. The drug storage container according to any one of claims 1 to 7, wherein the form of the drug or the auxiliary agent is a solid preparation.

9. The drug storage container according to claim 7 or 8, wherein
the closing member has a plug portion that is fitted into the opening portion of the container body, and
the drug or the auxiliary agent is retained on the plug portion.

10. The drug storage container according to claim 9, wherein
the plug portion of the closing member has a recess portion on a face crossing the fit-in direction, and
the drug or the auxiliary agent is retained on the recess portion.

11. A closing member, for use in the drug storage container according to any one of claims 8 to 10, being configured to detachably retain the solid preparation.

12. A method for manufacture of the drug storage container according to any one of claims 1 to 10, comprising:
a putting step of putting the drug through the opening portion into the container body; and
a closing step of closing the container body in a state where the auxiliary agent is retained on the closing member while placing a face of the closing member on which the auxiliary agent is retained at the opening portion side of the container body,
or comprising:
a putting step of putting the auxiliary agent through the opening portion into the container body; and
a closing step of closing the container body in a state where the drug is retained on the closing member while placing a face of the closing member on which the drug is retained at the opening portion side of the container body.

13. A method for manufacture of the drug storage container according to any one of claims 1 to 10, comprising:
a putting step of putting the drug in a liquid form through the opening portion into the container body;
a closing step of closing the container body in a state where the auxiliary agent is retained on the closing member while placing a face of the closing member on which the auxiliary agent is retained at the opening portion side of the container body; and
a drying step of lyophilizing the drug in a liquid form in the container body so that the drug is solidified, or comprising:
a putting step of putting the auxiliary agent in a liquid form through the opening portion into the container body;
a closing step of closing the container body in a state where the drug is retained on the closing member while placing a face of the closing member on which the drug is retained at the opening portion side of the container body; and
a drying step of lyophilizing the auxiliary agent in a liquid form in the container body so that the auxiliary agent is solidified.

14. A method for manufacture of the drug storage container according to claim 9 or 10, comprising:
a putting step of putting the drug in a liquid form through the opening portion into the container body;
a closing step of closing the container body by temporarily fitting the plug portion of the closing member to the opening portion of the container body in a state where the auxiliary agent is retained on the plug portion of the closing member, and
a drying step of lyophilizing the drug in a liquid form in the container body so that the drug is solidified in a temporarily fitted state;
or comprising:
a putting step of putting the auxiliary agent in a liquid form through the opening portion into the container body;
a closing step of closing the container body by temporarily fitting the plug portion of the closing member to the opening portion of the container body in a state where the drug is retained on the plug portion of the closing member, and
a drying step of lyophilizing the auxiliary agent in a liquid form in the container body so that the auxiliary agent is solidified in a temporarily fitted state.

15. The method for manufacture of a drug storage container according to claim 14, wherein
the plug portion of the closing member has the recess portion on a face crossing the fit-in direction,
the recess portion of the plug portion has an enough depth to expose a part of the recess portion through the opening portion of the container body when the plug portion is temporarily fitted to the opening portion of the container body in the closing step, and
the drug or the auxiliary agent is retained on the recess portion of the plug portion.

16. A microbial contaminant test method for testing a microbial contaminant using the drug storage container according to any one of claims 1 to 10, comprising:
a preparation step of putting an analyte into the container body of the drug storage container so that a sample to be subjected to measurement of a microbial contaminant, containing the drug, the auxiliary agent and the analyte, is prepared; and
a detection step of detecting the microbial contaminant in the sample.

17. The microbial contaminant test method according to claim 16, wherein the microbial contaminant is an endotoxin or a (1→3)-β-D-glucan.

18. A solid preparation for preparing a buffer solution, comprising at least a buffer and a molding agent, wherein
the buffer is contained in such an amount that the pH becomes neutral when the solid preparation is mixed with a predetermined aqueous solvent.

19. The solid preparation for preparation of a buffer solution according to claim 18, which does not substantially contain a microbial contaminant.
